(19) Europäisches Patentamt
European Patent Office
Office européen des brevets

(11) **EP 1 770 572 A2**

(12) **EUROPEAN PATENT APPLICATION**

(43) Date of publication:
**04.04.2007 Bulletin 2007/14**

(51) Int Cl.:
***G06F 19/00*** *(2006.01)*

(21) Application number: **06121279.1**

(22) Date of filing: **26.09.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **30.09.2005 JP 2005286030**

(71) Applicant: **OMRON CORPORATION**
**Kyoto-shi, Kyoto 600-8530 (JP)**

(72) Inventors:
• **ITO, Kazuo**
**KYOTO**
**Kyoto 600-8530 (JP)**
• **ANDO, Tanichi**
**KYOTO**
**Kyoto 600-8530 (JP)**

(74) Representative: **Weihs, Bruno Konrad et al**
**Osha Liang**
**121, Avenue des Champs Elysées**
**75008 Paris (FR)**

(54) **Information processing apparatus and information processing method, information processing system, program and recording media**

(57) The present invention discloses only required personal information in an emergency. A personal information display screen displays a face image display area 141, a passenger position display area 142, an interior image display area 143, a front image display area 144, a rear image display area 145, a personal information display area 146, and a display end button 147. For example, when an accident occurs and a rescue team arrives at the scene of the accident, a displ ay section of an on-vehicle apparatus displays not only passengers' face images but also required information such as their names, addresses, and blood types which is permitted to be disclosed. This enables emergencies to be dealt with both promptly and correctly even if such a serious accident as causes all the passengers to lose consciousness occurs. The present invention is applicable to the on-vehicle apparatus.

FIG. 1

**Description**

BACKGROUND OF THE INVENTION

Field of the Invention

The present invention relates to an information processing apparatus and information processing method, an information processing system, a program, and recording media, and in particular, to an information processing apparatus and information processing method, an information processing system, a program, and recording media which can be suitably used in an emergency such as an accident or a theft.

Description of the Related Art

In recent years, personal authentication techniques have been widely utilized to enable the identification of a person in a particular site in various opportunities. The personal authentication techniques include, for example, conventional magnetic and IC cards, a method of enabling authentication information to be transmitted and received in a non-contact manner on the basis of RFID or the like, and bio-authentication, which is now commonly used. In particular, a person in a particular site can be reliably identified using bio-authentication such as face image authentication, retina pattern authentication, or fingerprint authentication which uses personal biological features for authentication.

A driver's license personal recognition information managing system has been proposed in, for example, Japanese Patent Laid-Open No. 2003-108945. The system transmits ID information read from an ID chip in a driver's license, from an ID reading/displaying device to a diver's license managing center via a communication line. The system then reads, from a database in the driver's license managing center, personal recognition information on the holder of the driver's license corresponding to the ID information in the ID chip in the driver's license. The system then transmits the read personal recognition information on the holder of the driver's license, to the ID reading/displaying device via the communication line. The system thus causes the ID reading/displaying device to display the personal recognition information on the holder of the driver's license. This enables the holder of the driver's license to be authenticated on the basis of the ID information recorded in the ID chip in the driver's license. Consequently, when an accident occurs during driving, the personal recognition information on the holder of the driver's license can be immediately provided.

A technique has also been proposed which is used in emergency care to make it possible to provide a doctor or paramedic with medical information on a patient who is unconscious or has difficulty in responses. With this technique, when the patient suddenly feels sick, the patient himself or herself, in the case where he or she is conscious, or an arbitrary person present on the scene, in the case where the patient is unconscious, operates the patient's portable information terminal to transmit an emergency call from the portable information terminal to an emergency care information server. The emergency care information server communicates with an ambulance simultaneously with the reception of the emergency call or the portable information terminal communicates with a firehouse or the like simultaneously with the transmission of the emergency call. Thus, if the patient suddenly becomes worse, the single operation allows an ambulance or the like to be arranged for, while enabling identification information on that patient to be displayed on a screen of a cellular phone or the like (Japanese Patent Laid-Open No. 2003-162578).

Further, what is called a mayday service is provided. In a "state of emergency" such as an accident, a sudden illness, or a failure, depressing a predetermined button allows an operation center to notice the emergency without using a public telephone or an emergency telephone. For example, data such as GPS positional information is automatically transmitted to the operation center, which then communicates with the reporting person to check the situation and automatically or manually report it to a related organization such as the police or a firehouse via a network.

For example, if a passenger is authenticated using bio-authentication means such as face authentication or fingerprint authentication in order to manage the security of a car, a user authenticating apparatus in the car can record who got into or out of the car and when they got into or out of the car. However, in view of protection of personal information, it is not preferable that others can acquire an authentication result or personal information that can be acquired using the authentication result without permission or limitation.

If, for example, such a serious accident as causes all the passengers to lose consciousness occurs, it is necessary to quickly communicate with a rescue organization. However, the current mayday service does not allow the occurrence of an accident or personal information to be communicated to a rescue organization such as a firehouse if the user's intention to report it cannot be confirmed.

On the other hand, persons performing rescue operations on the scene of the accident can easily understand the emergency and thus report the accident to the police or a firehouse. However, these persons cannot determine who was in the vehicle causing the accident if, with the technique described in Japanese Patent Laid-Open No. 2003-108945, a driver's license with an IC chip has not been found or if, with the technique described in Japanese Patent Laid-Open No. 2003-162578, the patient's portable information terminal has not been found.

The present invention has been made in view of these circumstances. An object of the present invention is to, if a person is involved in an critical event such as an accident or a fire, allow others to access only appropriate parts of personal information relating to the event.

SUMMARY OF THE INVENTION

An information processing apparatus in accordance with an aspect of the present invention comprises authenticating means for authenticating a passenger in a vehicle, state managing means for managing a state of the vehicle, emergency determining means for determining whether or not the vehicle is in an emergency on the basis of the state of the vehicle managed by the state managing means, disclosed content determining means for determining a disclosed content of personal information on the passenger on the basis of the determination by the emergency determining means, personal information acquiring means for acquiring personal information on the passenger determined to be disclosed, on the basis of the determination by the disclosed content determining means, and disclosing means for disclosing the personal information on the passenger determined to be disclosed and which has been acquired by the personal information acquiring means.

This makes it possible to detect a defect in the vehicle to disclose personal information as required.

On this occasion, only the required personal information is disclosed.

The state managing means, emergency determining means, disclosed content determining means, and personal information acquiring means can be composed of, for example, a CPU or the like. The disclosing means can be composed of, for example, a display section, a voice output means, or a communication section.

The emergency determining means can further determine the level of the emergency if the vehicle is in the emergency, and the disclosed content determining means can determine the disclosed content of the personal information on the passenger on the basis of the determination of the level of the emergency by the emergency determining means.

This enables the contents of the disclosed personal information to be optimized on the basis of whether or not the emergency is serious.

The disclosed content determining means can further determine the disclosed content of the personal information on the passenger on the basis of a person who is referencing the personal information on the passenger disclosed by the disclosing means.

This makes it possible to, for example, prevent the personal information from being unnecessarily disclosed to a malicious person. The level of the disclosed information can be varied on the basis of whether the information is disclosed to the person himself or herself, his or her family member, or a third person or whether the rescuers are general persons or personnel such as paramedics or police officer who are assigned to a special mission.

The disclosed content determining means can further determine the disclosed content of the personal information on the passenger on the basis of settings present when the personal information on the passenger is registered.

The disclosed content determining means can further determine the disclosed content of the personal information on the passenger on the basis of the type of the vehicle.

For example, information to be disclosed when an accident or the like occurs varies depending on whether the vehicle is a bus, a train, or the like which has a public nature, or a passenger car. Accordingly, when the contents of personal information to be disclosed are determined on the basis of the type of the vehicle, it is possible to prevent more information than required from being disclosed to third persons.

The information processing apparatus can further comprise response confirming means for confirming a response from the passenger. The emergency determining means can determine whether or not the vehicle is in the emergency on the basis of the state of the vehicle managed by the state managing means and the response from the passenger confirmed by the response confirming means.

That is, if the vehicle is determined to be in the emergency on the basis of sensor inputs or image analysis and if the passenger does not give any response, the detection of the emergency is unlikely to be erroneous. It is also likely that a serious accident is occurring and that the passenger is unconscious.

The state managing means can manage the state of the vehicle on the basis of information on the state of the vehicle acquired by a sensor provided in the vehicle.

The state managing means can receive a supply of information indicative of the state of the vehicle acquired by a plurality of sensors provided in the vehicle, and the emergency determining means can determine whether or not an emergency is occurring on the basis of the state of the vehicle acquired by a plurality of the sensors.

This makes it possible to prevent the emergency from being erroneously detected as a result of the malfunctioning of the sensor.

The state managing means can manage the state of the vehicle on the basis of an image of an interior or exterior of the vehicle acquired by image acquiring means provided in the vehicle.

The image acquiring means is composed of, for example, a camera that uses a CMOS or a CCD.

The information processing apparatus can further comprise operation input means for receiving an input predetermined code number. The emergency determining means can recognize that the vehicle may have been stolen if the predetermined code number is input via the operation input means.

The operation input means is composed of various input devices, for example, a keyboard, buttons, a touch panel, and a mouse.

Thus, in an emergency such as a theft which cannot be detected by sensor inputs, only authorized persons (forexample, the persons concerned with the police) can confirm the registered holder of the vehicle.

The information processing apparatus can further comprise display means for displaying information. The disclosing means can control display, on the display means, of the personal information on the passenger determined to be disclosed which has been acquired by the personal information acquiring means.

The display means can display information containing a face image as personal information on the passenger.

Thus, even if, for example, a plurality of passengers lose consciousness owing to an accident, the rescuers can check the passengers against displayed photographs to correctly determine which of the passengers corresponds to the disclosed personal information.

The display means is composed of any of various display devices, for example, a liquid crystal display or a CRT display.

The information processing apparatus can further comprise display means for displaying information. The authenticating means can acquire a face image of the passenger and use the face image to execute an authenticating process, and the disclosing means can control the display, on the display means, of the personal information on the passenger determined to be displayed which has been acquired by the personal information acquiring means and the passenger's face image.

The information processing apparatus can further comprise display means for displaying information, and intention recognizing means for acquiring and analyzing the passenger's face image to recognize the passenger's intention. The disclosing means can control the display, on the display means, of the personal information on the passenger determined to be displayed which has been acquired by the personal information acquiring means and the passenger's face image acquired by the intention recognizing means.

This enables the various functions to cooperate with one another to provide an inexpensive, multifunctional on-vehicle apparatus.

The information processing apparatus can further comprise voice output means for outputting a voice. The disclosing means can control the notification, through the voice output means, of the personal information on the passenger determined to be disclosed which has been acquired by the personal information acquiring means.

The information processing apparatus can further comprise communication means for controlling transmission and reception of information to and from other apparatuses. The disclosing means can control the transmission, by the communication means, of the personal information on the passenger determined to be disclosed which has been acquired by the personal information acquiring means to the other apparatuses.

The communication means can use, for example, a short-distance radio communication function, a packet communication function, or the like to communicate with another apparatus.

The another apparatus may be, for example, a terminal apparatus held by the rescuer and having a short-distance radio communication function, or a server held by an insurance company, a security company, a vehicle distributor, the police, a firehouse, or a service provider for a reporting system in the emergency to which the present invention is applied or a managing organization that manages those who are registered with the system.

An aspect of the present inventionprovides an information processing method or program for an information processing apparatus that manages personal information on a passenger in a vehicle, the method comprising an authenticating step of authenticating a passenger in a vehicle, a state managing step of managing a state of the vehicle, an emergency determining step of determining whether or not the vehicle is in an emergency on the basis of the state of the vehicle managed by the state managing step, a disclosed content determining step of determining a disclosed content of personal information on the passenger on the basis of the determination by processing in the emergency determining step, a personal information acquiring step of acquiring personal information on the passenger determined to be disclosed, on the basis of the determination by processing in the disclosed content determining step, and disclosing step of disclosing the personal information on the passenger determined to be disclosed which has been acquired by processing in the personal information acquiring step.

This makes it possible to detect a defect in the vehicle to disclose personal information as required.

On this occasion, only the required personal information is disclosed.

An aspect of the present invention provides an information processing system comprising an on-vehicle apparatus mounted in a vehicle, and at least one information processing apparatus that transmits and receives information to and from the on-vehicle apparatus, the on-vehicle apparatus comprising authenticating means for authenticating a passenger in the vehicle, state managing means for managing a state of the vehicle, emergency determining means for determining whether or not the vehicle is in an emergency on the basis of the state of the vehicle managed by the state managing means, disclosed content determining means for determining a disclosed content of personal information on the passenger on the basis of the determination by the emergency determining means, personal information acquiring means for acquiring personal information on the passenger determined to be disclosed, on the basis of the determination by the disclosed content determining means, disclosing means for disclosing the personal information on the passenger determined to be disclosed which has been acquired by the personal information acquiring means, and communication means that transmits and receives information to and from the information processing apparatus.

The state managing means, emergency determining means, disclosed content determining means, and personalinformation acquiring means can be composed of, for example, a CPU or the like. The disclosing means can be composed of, for example, a display section, a voice output section, or a communication section.

The communication means can use, for example, a short-distance radio communication function, a packet communication function, or the like to communicate with an information processing apparatus.

The information processing apparatus included in the information processing system may be, for example, a terminal apparatus held by the rescuer and having a short-distance radio communication function, or a server held by an insurance company, a security company, a vehicle distributor, the police, a firehouse, or a service provider for a reporting system of an emergency to which the present invention is applied or a managing organization that manages those who are registered with the system.

If the personal information on the passenger is transmitted to the information processing apparatus by the communication means controlled by the disclosing means, the disclosed content determining means determines the disclosed content of the personal information on the passenger on the basis of the type of the information processing apparatus to which the personal information on the passenger is disclosed.

Thus, if the destination of the personal information is a server managed by a firehouse or the like, medical information is disclosed. If the destination of the personal information is a server managed by the police or the like, personal information such as the address and name and the address of a person to communicate with in an emergency is disclosed. If the destination of the personal information is a server managed by an insurance company, a security company, or the like, personal information corresponding to the contents of contracted services is disclosed.

The information processing apparatus stores the personal information on the passenger, and the personal information acquiring means controls the communication means to acquire the personal information on the passenger from the information processing apparatus.

This makes it possible to acquire and disclose personal information on a passenger whose personal information is not registered in the vehicle. The personal information on the passenger can be acquired and disclosed even if the personal information registered in the vehicle cannot be read for any reason.

According to an aspect of the present invention, the passenger in the vehicle is authenticated and the state of the vehicle is managed. The apparatus or method determines whether or not the vehicle is in the emergency on the basis of the state of the vehicle. The apparatus or method further determines on the basis of the determination the disclosed content of the personal information on the passenger. The apparatus or method then acquires and discloses the personal information on the passenger determined to be disclosed.

This enables persons such as rescuers who deal with the emergency to acquire information required to deal with the emergency on the scene without any special equipment. It is thus possible to deal with the situation both appropriately and promptly while avoiding the leakage of more secrete information than required.

As described above, an aspect of the present invention enables required personal information to be disclosed when an emergency occurs. In particular, persons such as rescuers who deal with the emergency can acquire personal information required to deal with the emergency on the scene without any special equipment. It is thus possible to deal with the situation both appropriately and promptly while avoiding the leakage of more secrete information than required.

BRIEF DESCRIPTION OF THE DRAWINGS

FIG. 1 is a block diagram showing the configuration of an on-vehicle apparatus to which the present invention is applied;
FIG. 2 is a block diagram illustrating the configuration (functions) of an authentication processing section;
FIG. 3 is a diagram illustrating a registration screen;
FIG. 4 is a block diagram illustrating the configuration (functions) of an emergency detecting section;
FIG. 5 is a block diagram illustrating the functions of a CPU;
FIG. 6 is a diagram illustrating a personal information display screen;
FIG. 7 is a block diagram showing an embodiment of an intention checking apparatus;
FIG. 8 is a schematic diagram showing the directions of face and line of sight of a person looking forward;
FIG. 9 is a schematic diagram showing the condition of the eyes of the person shown in FIG. 8;
FIG. 10 is a schematic diagram showing the directions of face and line of sight of a nodding person;

FIG. 11 is a schematic diagram showing the condition of the eyes of the person shown in FIG. 10;

FIG. 12 is a graph illustrating the case in which the likelihood of nodding is at least a threshold;

FIG. 13 is a schematic diagram showing the directions of face and line of sight of a person looking forward;

FIG. 14 is a schematic diagram showing the condition of the eyes of the person shown in FIG. 13;

FIG. 15 is a schematic diagram showing the directions of face and line of sight of a person shaking his or her head;

FIG. 16 is a schematic diagram showing the condition of the eyes of the person shown in FIG. 15;

FIG. 17 is a graph illustrating the case in which the likelihood of head shaking is at least anupper-limit threshold and at most a lower-limit threshold;

FIG. 18 is a flowchart illustrating an emergency monitoring process;

FIG. 19 is a flowchart illustrating an access prohibition canceling process 1;

FIG. 20 is a diagram illustrating an emergency personal information providing system to which the present invention is applied;

FIG. 21 is a block diagram showing the configuration of an on-vehicle apparatus shown in FIG. 20;

FIG. 22 is a block diagram illustrating the functions of a CPU;

FIG. 23 is a diagram illustrating a personal information display screen;

FIG. 24 is a flowchart illustrating an access prohibition canceling process 2; and

FIG. 25 is a flowchart illustrating an external notification process.

Description of Symbols

| | |
|---|---|
| 1 | On-vehicle apparatus |
| 11 | CPU |
| 14 | Operation input section |
| 15 | Biological information acquiring section |
| 16 | Authentication processing section |
| 17 | Authentication result storing section |
| 18 | Personal information storing section |
| 19 | Emergency detecting section |
| 20 | External operation section |
| 21 | Display section |
| 22 | Voice output section |
| 23 | Communication section |
| 121 | Authentication result acquiring section |
| 122 | State managing section |
| 123 | Response processing means |
| 124 | Notification control section |
| 125 | Emergency level determining section |
| 126 | Personal information access level determining section |
| 127 | Personal information acquiring section |
| 128 | External operation control section |
| 271 | On-vehicle apparatus |
| 251 | Radio packet communication network |
| 251 | Internet |
| 282 | Personal information registering server |
| 283 | Monitoring server |
| 284 | Emergency managing server |
| 285 | Accident managing server |
| 286 | Client Managing server |
| 291 | CPU |
| 292 | Communication section |
| 301 | Personal information access level determining section |
| 302 | Notification control section |

DETAILED DESCRIPTION OF THE PREFERRED EMBODIMENTS

Embodiments of the present invention will be described below with reference to the drawings.

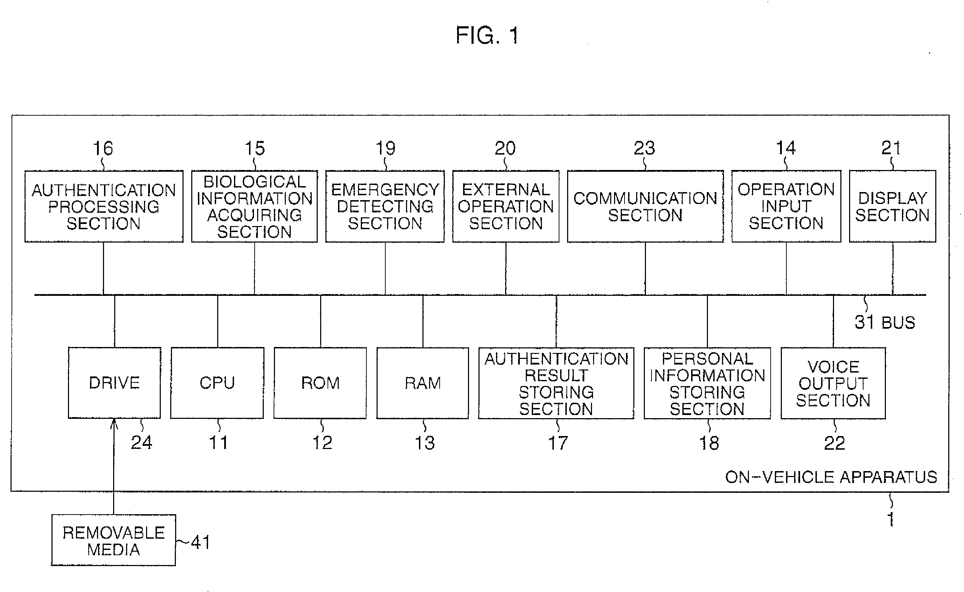

FIG. 1 shows the configuration of an on-vehicle apparatus.

The on-vehicle apparatus 1 is mounted in, for example, a passenger car, a bus, a train, or the like. Personal information on passengers can be registered in the on-vehicle apparatus 1. The on-vehicle apparatus 1 can also execute a process of authenticating passengers.

The on-vehicle apparatus 1 comprises a CPU 11, a ROM 12, a RAM 13, an operation input section 14, a biological information acquiring section 15, an authentication processing section 16, an authentication result storing section 17, a personal information storing section 18, an emergency detecting section 19, an external operating section 20, a display section 21, a voice output section 22, and a communication section 23. The on-vehicle apparatus 1 further comprises a drive 24 as required. These components are interconnected via a bus 31.

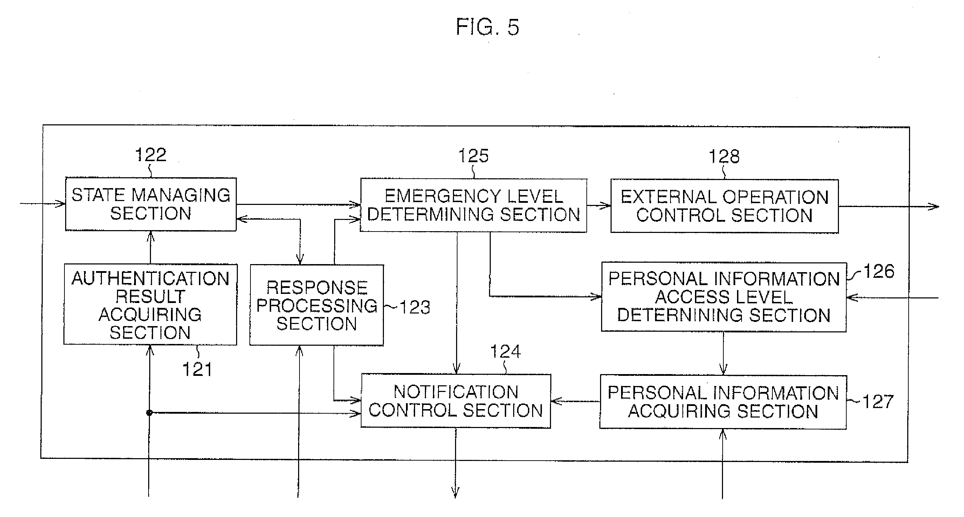

The CPU 11 controls the operation of each section of the on-vehicle apparatus 1. With reference to FIG. 5, a detailed description will be given of functions realized by the CPU 11 expanding predetermined application programs stored in the ROM 12 into the RAM 13 for execution.

The ROM 12 appropriately stores the application programs executed by the CPU 11 and data required to execute the application programs. The RAM 13 appropriately stores data required for the CPU 11 to execute various processes.

The operation input section 14 is composedof, for example, a touch panel, buttons, keys, levers, a keyboard, a mouse, and a receiving section that receives infrared signals transmitted by a remote commander (not shown). The operation input section 14 receives and supplies a user's operation input to the CPU 11 via the bus 31.

The operation input section 14 receives an input code number or the like which is known only to the police, in an emergency such as a theft of a vehicle which is not detected by the emergency detecting section 19.

The biological information acquiring section 15 includes a camera to acquire, for example, a face, fingerprint, retina, or vein image as well as information required to identify a person. For example, the biological information acquiring section 15 uses a motion picture camera with a built-in CCD or CMOS image pickup element to acquire a face image of a passenger. The biological information acquiring section 15 then supplies the face image to the authentication processing section 16 via the bus 31. In the description of the present embodiment, the biological information acquiring section 15 acquires a face image as biological information. However, the biological information acquiring section 15 may acquire arbitrary biological information.

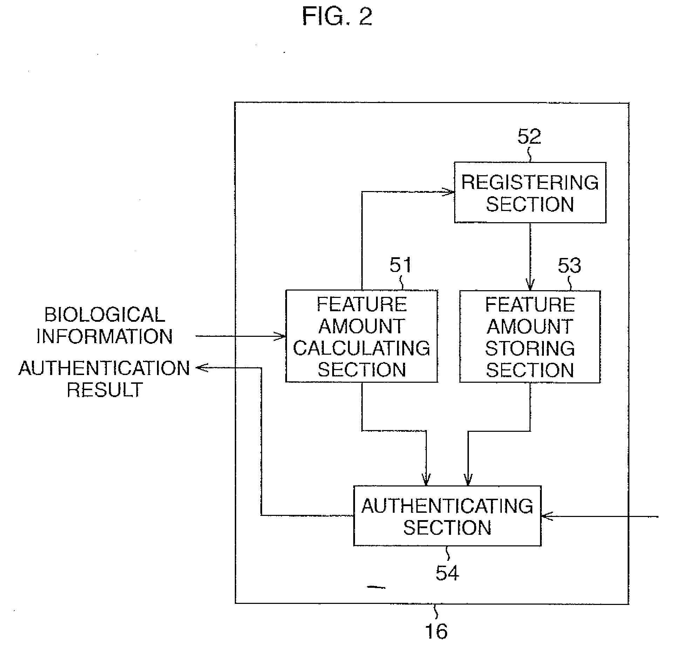

The authentication processing section 16 authenticates a person in a vehicle or the like in which the on-vehicle apparatus 1 is mounted, on the basis of the biological information acquired by the biological information acquiring section 15. An example of configuration of the authentication processing section 16 will be described below in detail with reference to FIG. 2.

The authentication result storing section 17 stores the result of an authentication executed by the authentication processing section 16.

The personal information storing section 18 stores personal information. The personal information stored in the personal information storing section 18 includes the user's face image acquired by the biological information acquiring section 15 and various information input by the operation input section 14. A method of registering personal information will be described below.

The authentication result storing section 17 and the personal information storing section 18 are composed of, for example, nonvolatile semiconductor memories or hard disks. The authentication result storing section 17 and the personal information storing section 18 maybe individually constructed or may be composed of the same storage media having their own storage areas.

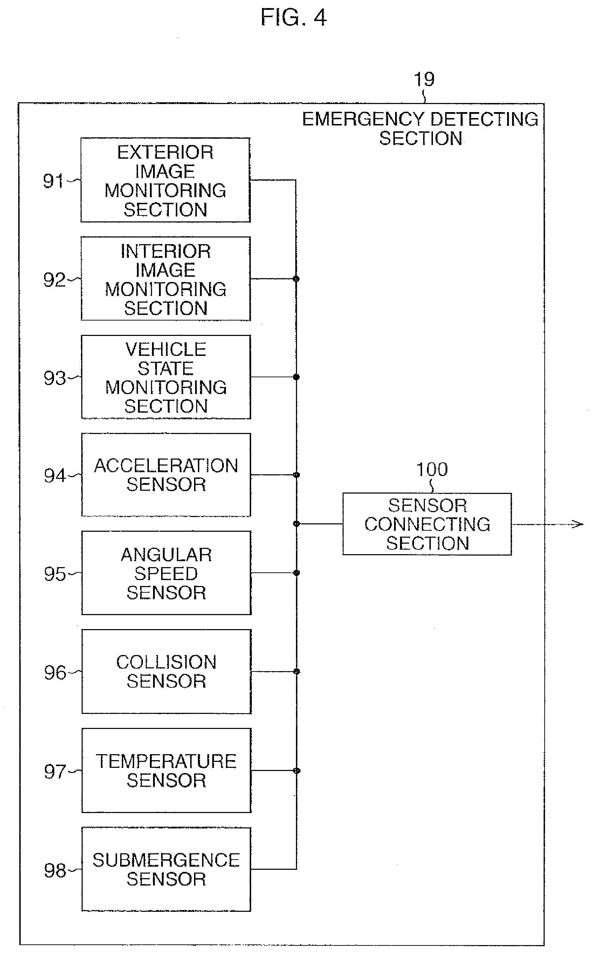

The emergency detecting section 19 detects an emergency such as an accident which occurs to, for example, a passenger, a bus, or the like in which the on-vehicle apparatus is mounted. An example of configuration of the emergency detecting section 19 will be described below in detail with reference to FIG. 4.

The external operation section 20 controls a key, a motor, and other actuators as required for the emergency under the control of the CPU11; when the on-vehicle apparatus 1 is mounted in a passenger car, the external operation section 20 unlocks the key to the doors, or when the on-vehicle apparatus 1 is mounted in a bus, a train, or the like, the external operation section 20 automatically opens the doors, which is otherwise controllably opened or closed by a driver.

The display section 21 is composed of, for example, a display such as a CRT (Cathode Ray Tube) or an LCD (Liquid Crystal Display) and a driver that controls the display of images or letters on the display. Under the control of the CPU 11, the display section 21 displays messages for the user, a GUI (Graphical User Interface) allowing the user to register information required for an authenticating process, or an authentication process result or personal information on registered passengers which is displayed in an emergency.

The voice output section 22 is composed of a speaker to output messages for the user or a voice notifying the user of an authentication process result, or a voice checking the user for consciousness in an emergency, under the control of the CPU 11.

The communication section 23 is connected to a wide area network such as the Internet by wire or radio or to a radio telephone network to control the transmission and reception of information to and from external apparatuses.

The drive 24 is connected to the bus 31 as required. Removable media 41 such as a magnetic disk, optical disk, magneto optic disk, or semiconductor memory is appropriately installed in the bus 31. A computer program read from the removable media 41 is installed in a storing section such as the RAM 13 or a hard disk (not shown) as required.

FIG. 2 shows an example of detailed configuration of the authentication processing section 16.

In the description below of the authentication processing section 16 in FIG. 2, the biological information acquiring section 15 acquires a face image as biological information in this case, and the authentication processing section 16 calculates the feature amount of the face image acquired to execute an authenticating process. However, the biological information acquiring section 15 may acquire biological information different from the face image and the authentication processing section 16 may execute an authenticating process on the basis of the biological information acquired.

The authentication processing section 16 is composed of a feature amount calculating section 51, a registering section 52, a feature amount storing section 53, and an authenticating section 54.

In a registration mode, the feature amount calculating section 51 extracts a feature amount from biological information (for example, a face image) on a registered person supplied by the biological information acquiring section 15 and supplies it to the registering section 52. In an authentication mode, the feature amount calculating section 51 extracts the feature amount from the biological information (for example, a face image) on the registered person supplied by the biological information acquiring section 15 and supplies it to the authenticating section 54.

In the registration mode, the registering section 52 registers the feature amount of the biological information (for example, a face image) supplied by the feature amount calculating section 51, in the feature amount storing section 53.

The feature amount storing section 53 stores the feature amount used for an authenticating process executed by the authenticating section 54.

In the authentication mode, the authenticating section 54 compares the feature amount of the biological information (for example, a face image) on the passenger supplied by the feature amount calculating section 51 with the feature amount of the biological information (for example, a face image) on the registered persons stored in the feature amount storing section 53. The authenticating section 54 thus determines whether or not the passenger to be authenticated is registered, and if the passenger is registered, which of the registered persons the passenger is.

The authenticating section 54 executes an authenticating process for each of the seats in the vehicle with the on-vehicle apparatus 1. The authenticating process for each seat may be executed, for example, when opening or closing of doors of the vehicle is detected or when movement of a person is detected in an aisle in the vehicle or the like or every predetermined time. Alternatively, for example, images of the seats may be always monitored so that the authenticating process for each seat can be executed when seating of a new person is detected. In a vehicle with the on-vehicle apparatus 1, if the driver's face image is always acquired in order to check the driver's intention as described below, an authenticating process may of course be executed utilizing the face image.

The authenticating section 54 is supplied with information that can be used for an authenticating process together with the biological information, for example, a code number input by the user via the operation input section 14 and supplied via the bus 31, information contained in an IC card and read by an IC card reader (not shown), or an ID contained in an RFID tag and read by a dedicated receiving device. The authenticating section 54 executes a combination of authentication based on biological information and authentication based on other information. In this case, the authenticating section 54 has information required for an authenticating process such as code numbers or IDs registered in itself.

The method of identifying a person using face images may use any of the above methods.

Instead of the authentication processing section 16, a software program providing functions described with reference to FIG. 2 may be stored in the ROM 12; the software program is expanded into the RAM 13 and executed by the CPU 11.

Now, description will be given of registration and updating of personal information stored in the personal information storing section 18, taking the case in which the on-vehicle apparatus 1 is mounted in a passenger car as an example.

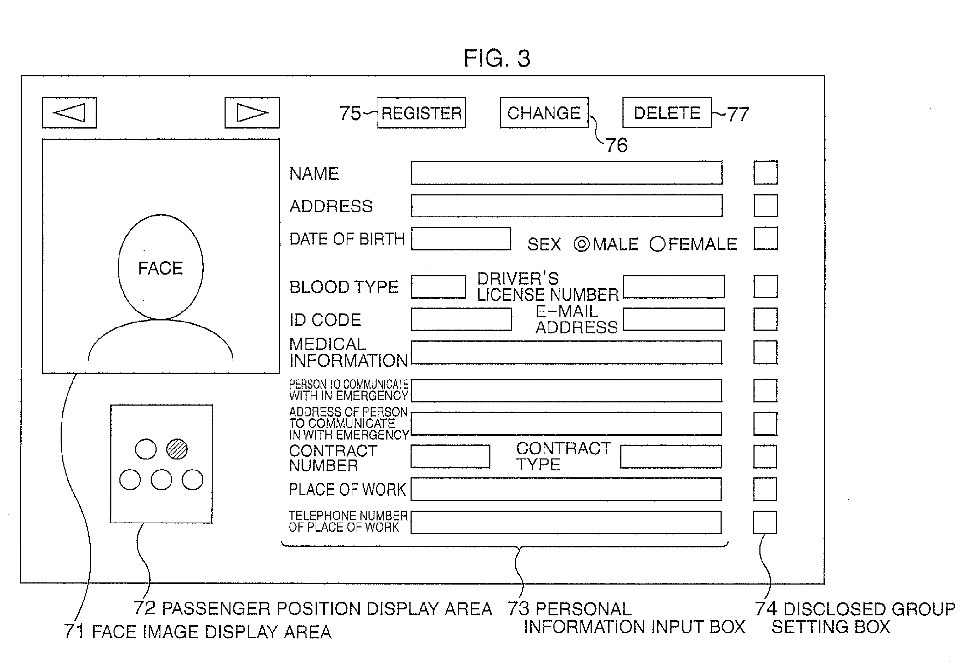

When an instruction to start a personal registration mode is input via the operation input section 14, a registration screen such as the one shown in FIG. 3 is displayed on the display section 21 in the registration mode.

The registration screen displays a face image display area 71, a passenger position display area 72, personal information input box 73, a disclosed group setting box 74, a registration button 75, a change button 76, and a delete button 77. A registration screen similar to that displayed for a new registration process is displayed to allow the user to change registered information.

The face image display area 71 displays, in the registration mode, a face image of a registered person acquired by the camera provided in the biological information acquiring section 15.

The passenger position display area 72 shows the position of a passenger being registered. In FIG. 3, the on-vehicle apparatus 1 is mounted in a 5-passenger car. However, if the on-vehicle apparatus 1 is mounted in a passenger car in a different form or a different vehicle, the display of the passenger position display area 72 is differently configured from that in FIG. 3 so as to conform to the vehicle or the like with the on-vehicle apparatus 1.

The personal information input box 73 displays personal information on a registered person which has been input via the operation input section 14 in the registration mode. The user can register, for example, his or her name, address, date of birth, sex, blood type, driver's license number, ID code normally required to access the personal information (which may be issued to the user owning the on-vehicle apparatus 1 or which may be set by the user in registering the personal information), and e-mail address, and, for example, medical information such as allergy, contraindication, or medical history which is considered to be critical in an emergency, information on a person to communicate with in an emergency, a contact number or type such as a car insurance, and the organization to which the user or the vehicle belongs or its telephone number. The contents of registration in the personal information input box 73 in FIG. 3 are only illustrative. Other information can of course be registered, for example, whether the registered person is registered as a donor.

If the level of disclosed information is varied depending on, for example, the level of emergency when a registered person's personal information to be displayed in a personal information input box 73 is disclosed, the disclosed group setting box 74 is used to set a disclosed group and the level of disclosed information. For example, if it is necessary to check whether or not a vehicle or the like with the on-vehicle apparatus 1 has been stolen, the blood type, medical information, or e-mail address need not be disclosed. Instead, only the registered person's face image is displayed to allow the user to check whether or not the current driver is the correct owner. However, if the vehicle or the like with the on-vehicle apparatus 1 is involved in an accident, it is suitable to disclose personal information corresponding to the level of the accident. If the on-vehicle apparatus 1 is mounted in a public means of transportation such as a bus, personal information such as the name or address is desirably prevented from being disclosed to third persons without limitation except in an emergency. Accordingly, the disclosed group and the level of disclosed information are set in the disclosed group setting box 74 depending on the type of the vehicle with the on-vehicle apparatus 1, the situation in which the registered personal information is assumed to be utilized, and persons who reference the registered personal information.

The registration button 75 is selected (depressed if the display section 21 and the operation input section 14 constitutes a touch panel) by a registered person to register set contents in the personal information storing section 18 after checking information set via the personal information input box 73 and disclosed group setting box 74.

The change button 76 is selected or depressed to read and display personal information registered and stored in the personal information storing section 18 to change registered contents.

The delete button 77 is selected or depressed to delete information set via the personal information input box 73 or disclosed group setting box 74 or registered information read from the personal information storing section 18.

If, for example, the on-vehicle apparatus 1 is mounted in a general passenger car, then in the registration mode, the camera provided in the biological information acquiring section 15 is used to acquire face images of registered persons in a predetermined order, that is, starting with the driver, followed by the person next to the driver and then the passengers in the rear seat. The face images are then displayed in the face image display area 71. Personal information on the corresponding registered persons is then registered via the personal information input box 73 and disclosed group setting box 74 and stored in the personal information storing section 18.

The personal information on the thus registered persons is stored in the personal information storing section 18. The personal information on a registered person is normally accessible only when that person is authenticated by an authenticating process using biological information (face image) or when his or her personal ID is input. In other words, the personal information on a registered person is accessible to that person or a person permitted by the registered person (who is permitted to know the registered person's personal ID). If a high level of emergency is detected, the prohibition of accesses to the personal information is cancelled to allow the personal information corresponding to the emergency level to be externally accessed or to be automatically disclosed.

The registered personal information is associated with the result of personal authentication executed by the authentication processing section 16 to identify a passenger.

Specifically, the authentication processing section 16 executes personal authentication to identify the passenger in each seat of the vehicle with the on-vehicle apparatus 1. If the authentication result storing section 17 stores the authentication result for the passenger in each seat, the personal information stored in the personal information storing section 18 is associated with the authentication result stored in the authentication result storing section 17, so that when an emergency occurs, the personal information on the passenger authenticated to be in each seat is quickly presented.

If the on-vehicle apparatus 1 is mounted in a vehicle serving as a public means of transportation, for example, a bus or a train, biological information on passengers, for example, their face images, is acquired when they get on the vehicle. For example, personal information which is similar to that described above and which is stored in electronic tickets, cellular phones, or RFIDs may be acquired. Then, the acquired personal information is associated with the biological information such as face images and stored in the personal information storing section 18. The personal information registered when the passengers get on the vehicle may be erased when the corresponding registered persons are detected to have gotten off the vehicle.

FIG. 4 shows an example of detailed configuration of the emergency detecting section 19 in detail.

The emergency detecting section 19 includes any or a combination of, for example, an exterior image monitoring section 91, an interior image monitoring section 92, a vehicle state monitoring section 93, an acceleration sensor 94, an angular speed sensor 95, a collision sensor 96, a temperature sensor 97, and a submergence sensor 98. The emergency detecting section 19 is connected to the bus 31 via a sensor connecting section 100.

The exterior image monitoring section 91, interior image monitoring section 92, and vehicle state monitoring section 93 may be installed in the form of software that processes images from image pickup means (not shown)

The exterior image monitoring section 91 processes any or a combination of images of the exterior picked up by the image pickup means (not shown) picking up images of the front, rear, and surroundings of the vehicle. The exterior image monitoring section 91 thus detects an emergency such as a collision against an obstacle, another car, a motorbike, a bicycle, a pedestrian, or an animal, overturning, or tumbling-down. For example, the exterior image monitoring section 91 can process an image of front of the vehicle to detect an emergency such as a collision against another car, an obstacle, a guardrail, or a pedestrian. The exterior image monitoring section 91 can also process an image of rear of the vehicle to detect a collision during backup or a collision of another vehicle. The exterior image monitoring section 91 can process an image of surroundings of the vehicle to detect hoodlums destroying the windows using tools or iron pipes.

The interior image monitoring section 92 processes an image of the interior picked up by the image pickup means (not shown) to monitor the state of the passengers and objects in the vehicle. The interior image monitoring section 92 detects an emergency such as a collision of a passenger against belongings, the car body, or another passenger which affects the passenger.

When the authentication processing section 16 executes personal authentication using face images as described above, the authentication may be executed using an image of the interior acquired by the interior image monitoring section 92. By executing an authenticating process using an image of the interior, it is possible to detectwhichof the registered persons is in the image and where that person is seated in the vehicle.

The vehicle state monitoring section 93 acquires information on the state of the vehicle, for example, a car speed, a car acceleration, a steering operation, or a brake operation, which is used to monitor the state of the vehicle before the occurrence of an accident on the basis of information required to control actuators for the vehicle with the on-vehicle apparatus 1, or sensors or driving sections provided in the actuators.

A predicted value for damage to a passenger can be calculated on the basis of the information on the vehicle state which is acquired by the vehicle state monitoring section 93 if, for example, an accident occurs.

The acceleration sensor 94 acquires the acceleration of the vehicle with the on-vehicle apparatus 1 to detect, for example, an acceleration that is impossible under normal traveling conditions. The acceleration sensor 94 can thus detect an emergency such as a collision.

The angular speed sensor 95 acquires an angular speed observed if the vehicle with the on-vehicle apparatus 1 is tilted to detect, for example, an angular speed that is impossible under normal traveling conditions. The angular speed sensor 95 can thus detect a vehicle state that seriously affects the passengers, such as rolling or upsetting of the vehicle. The angular speed sensor 95 may include a rate sensor sensing the turning angle (angular speed) of the vehicle which can be used to sense the stability of the vehicle during cornering or to predict the route of the vehicle. The angular speed sensor 95 detects an angular speed that is impossible under normal traveling conditions, thus making it possible to detect an emergency such as spinning of the vehicle.

The acceleration information acquired by the acceleration sensor 94 enables the calculation of the level of damage to a passenger in an accident. The angular speed information acquired by the angular speed sensor 95 enables the detection of the vehicle state seriously affecting the passengers, such as rolling or upsetting of the vehicle.

The collision sensor 96 is integrated into, for example, a bumper or a side of the vehicle to detect deformation caused by a collision.

The temperature sensor 97 detects a high temperature resulting form the extraordinary temperature of any part or a fire.

The submergence sensor 98 detects that the vehicle has submerged on the basis of, for example, a change in the electric resistance between wires.

Various well-known techniques are available for assuming damage to the vehicle or passengers using the above sensors if an accident occurs. The on-vehicle apparatus 1 can use any or a combination of these techniques.

The sensor connecting section 100 supplies information acquired by the exterior image monitoring section 91 to submergence sensor 98, to the CPU 11 via the bus 31.

FIG. 5 is a functional block diagram illustrating functions provided by the CPU 11 executing predetermined programs.

The functions (function modules) provided by the CPU 11 executing predetermined programs include an authentication result acquiring section 121, a state managing section 122, a response checking section 123, a notification control section 124, an emergency level determining section 125, a personal information access level determining section 126, a personal information acquiring section 127, and an external operation control section 128.

The authentication result acquiring section 121 acquires the result of an authentication process executed by the authenticating processing section 16, the result being stored in the authentication result storing section 17.

The state managing section 122 manages the state of the vehicle with the on-vehicle apparatus 1 on the basis of an emergency detected by the emergency detecting section 19 or the user's operation input supplied by the operation input section 14. The state managing section 122 suitably determines whether or not the vehicle is in an emergency on the basis of a plurality of parameters detected by the plurality of sensors provided in the emergency detecting section 19 instead of a single parameter detected by one of the sensors. This prevents the situation in which, for example, an emergency is erroneously detected as a result of a failure in any of the various sensors provided in the emergency detecting section 19 so that the personal informal ion is unnecessarily disclosed.

The response checking section 123 controls the notification control section 124 as required under the control of the state managing section 122 so that the notification control section 124 communicates an aural or visual message to the user in order to determine the level of the emergency. The response processing means 123 thus checks whether or not the user has responded to the message or the contents of the user's response, on the basis of the user's operation input supplied by the operation input section 14.

Specifically, if the state managing section 122 detects the possibility of an emergency, the response checking section 123 controls a process of checking whether or not the passenger is safe by giving him or her a voice or screen message, for example, "Are you all right? Would you mind disclosing personal information such as medical information to the rescuers?". The response checking section 123 checks whether or not the user has responded on the basis of voice recognition or an operation input via the touch panel or button. If the passenger has not responded in spite of the detection by the state managing section 122 of the possibility of an emergency, the response checking section 123 determines that the passenger has lost consciousness or cannot respond owing to physical restrictions. The emergency level determining section 125 thus determines that the level of the emergency is high.

The notification control section 124 controls the display section 21 or the voice output section 22 as follows. An aural or visual message is communicated to the user in order to determine the level of the emergency under the control of the response checking section 123 or the information on the authenticatedperson (who is in the vehicle with the on-vehicle apparatus 1) is displayed in accordance with the emergency level determination and personal information access level determination under the control of the emergency level determining section 125.

The emergency level determining section 125 determines the level of the emergency on the basis of the state of the vehicle with the on-vehicle apparatus 1, which is managed by the state managing section 122, whether or not the user has responded to a message, which is checked by the response checking section 123, or the contents of the user's response.

The personal information access level determining section 126 determines the level of accesses to the personal information on the basis of the emergency level determination made by the emergency level determining section 125 and on the basis of an operation input from a person requesting an access to the personal information which input is supplied by the operation input section 14, as required. The personal information access level determining section 126 then controls acquisition of the personal information by the personal information acquiring section 127.

The personal information acquiring section 127 acquires the personal information permitted to be accessed, from the personal information storing section 18 under the control of the personal information access level determining section 126 and supplies it to the communication control section 124 for display.

The external operation control section 128 controls the external operation section 20 on the basis of the emergency level determination made by the emergency level determining section 125 so that the external operation section 20 operates the key, motor, or other actuators to deal with the emergency. For example, when the on-vehicle apparatus 1 is mounted in a passenger car, the external operation section 20 unlocks the key to the doors, or when the on-vehicle apparatus 1 is mounted in a bus, a train, or the like, the external operation section 20 automatically opens the doors, which is otherwise controllably opened or closed by the driver made by the emergency level determining section 125.

For example, when the rescue team approaches the vehicle, they cannot promptly rescue the passengers if the doors remain locked. The external operation control section 128 thus outputs an instruction to unlock the doors on the basis of the emergency level determination made by the emergency level determining section 125.

The doors maybe unlocked only if the approaching rescuers are confirmed to be rescue team members, police officers, or guards from a contracted security company by allowing the exterior image monitoring section 92 to check an exterior image or checking whether or not they have predetermined RFID tags or if they are confirmed not to be covering their faces by allowing the exterior image monitoring section 92 to check the exterior image. That is, the doors may be unlocked only if it can confirmed that the rescuers are successfullybehaving out of good will; they are not suspicious persons approaching the vehicle out of malice. Alternatively, the doors may be unlocked when, for example, the exterior image monitoring section 92 has acquired face images of the rescuers.

Any of the storing sections such as the RAM 13 suitably provides a function of allowing a list of suspicious persons to be registered in the storing section and managing the list. Then, a particular person, for examples, a stalker, may be registered in the list of suspicious persons so that the doors are not unlocked if the listed person is detected.

The doors may be unlocked on the basis of the contents of the emergency while omitting the authentication of approaching persons such as rescuers. Specifically, the doors may be immediately unlocked if the occurrence of a fire is detected.

Now, description will be given of an example of detection of an emergency and operations performed after the detection.

If the emergency level detecting section 19 detects an emergency, for example, a collision, turning of the vehicle, or a sudden, high acceleration in a direction different from the advancing direction, the state managing section 122 determines the possibility of a collision to allow the response processing section 123 to provide a message asking the passengers' intention.

The response processing section 123 controls the notification control section 124 so that an aural or visual message is given to the users in order to determine the level of the emergency. The notification control section 124 controls the display section 21 or the voice output section 22 so that the display section 21 or the voice output section 22a outputs a visual or aural message asking the users' intention, for example, "Are you all right? Would you mind disclosing personal information such as medical information to the rescuers?". If the emergency is critical, for example, the leakage of fuel is detected, the response processing section 123 suitably controls the notification control section 124 so that the notification control section 124 outputs a message notifying the users of a danger, for example, "Fuel is leaking. A fire may break out. Stop the engine and get out of the car."

The response processing section 123 notifies the emergency level determining section 125 whether or not any of the passengers has responded.

The emergency level determining section 125 determines the emergency level on the basis of the result of detection of the emergency supplied by the state managing section 122 as well as whether or not any of the passengers has responded. Specifically, if any passenger has responded, the emergency level determining section 125 determines that the level of the detected emergency ranks lower among the emergencies the levels of which are determined on the basis of result of detection of the emergency. If no passengers have responded, the emergency level determining section 125 determines that the level of the detected emergency ranks higher among the emergencies the levels of which are determined on the basis of result of detection of the emergency.

If the emergency level determining section 125 determines that the emergency level is high, the external operation control section 128 controls the external operation section 20 to cause it to, for example, unlock the key to the vehicle or automatically open the doors, so that the passengers can promptly get out of the car to take refuge or rescuers can check the interior of the vehicle to rescue the passengers as required.

The personal information access level determining section 126 determines the level of accesses to the disclosed personal information on the basis of the emergency level determination made by the emergency level determining section 125 and on the basis of an operation input from a person requesting an access to the personal information which input is supplied by the operation input section 14, as required.

As described above, the personal information registered in the on-vehicle apparatus 1 is normally accessible only to the registered persons and particular permitted persons. However, if the vehicle with the on-vehicle apparatus 1 becomes abnormal and a high emergency level is detected, outsiders can access the otherwise inaccessible personal information.

The personal information is desirably disclosed on the basis of the contents of the emergency as a result of the cancellation of prohibition of accesses. The personal information is also desirably disclosed only to persons identified in a certain manner (for example, police officers, the ambulance crew, or persons whose face images have been acquired) instead of an unspecified number of people as a result of the cancellation of access prohibition.

The person requesting an access to the personal information (or checking the displayed personal information) can suitably be identified when a predetermined code number is input via the operation input section 14. The person requesting an access to the personal information can also suitably be identified by analyzing an image acquired by the exterior image monitoring section 91 of the emergency detecting section 19. Alternatively, rescuers equipped with particular RFID tags may be authenticated to be permitted to acquire the personal information. The access prohibition may be cancelled when the exterior image monitoring section 91 of the emergency detecting section 19 has acquired face images of the rescuers.

The access level of the personal information determined by the personal information access level determining section 126 can be determined depending on the disclosed group set at the time of registration and the person to whom the information is disclosed. For example, a registered person can set the disclosed group at the time of registration as follows: the information for which a disclosed group A is set at the time of registration is disclosed to rescuers, the information for which a disclosed group B is set at the time of registration is disclosed only to the family members, and the information for which a disclosed group C is set at the time of registration is disclosed only to the registered person.

The disclosed information may be selected depending on the emergency level. For example, if it is necessary to check whether or not the vehicle or the like with the on-vehicle apparatus 1 has been stolen and if only the face information on the registered person has been disclosed, the blood type, medical information, or e-mail address need not be disclosed. However, if the vehicle or the like with the on-vehicle apparatus 1 is likely to have been involved in an accident, the blood type, the medical information, and the address of the person to communicate with in an emergency need to be disclosed.

The disclosed information may also be selected depending on both the emergency level and the person accessing the personal information. If, for example, the emergency level is very high but the person accessing the personal information cannot be identified, it is possible to disclose important, accessible medical information such as the position of each passenger in the vehicle and the passenger's face and blood type (that is, the information identifying the passenger such as the name and address is not disclosed and only the medical information is disclosed which is unlikely to be abused even when disclosed but which is required if the passenger is expected to be seriously injured and thus undergo emergency treatment).

The disclosed information may also be selected on the basis of both the type of the vehicle with the on-vehicle apparatus 1 and the emergency level. For example, if the vehicle with the on-vehicle apparatus 1 is a public means of transportation such as a bus, the personal information such as the name and address is desirably prevented from being disclosed to third persons without limitation as far as possible, except in an emergency.

The personal information access level determining section 126 may comprise a function of referencing the above list of suspicious persons. Then, a particular person, for examples, a stalker, maybe registered in the list of suspicious persons so that if the listedperson is detected to be requesting an access to the personal information, it is possible to inhibit the cancellation of prohibition of accesses to the personal information.

This configuration enables only the required information to be disclosed while preventing unnecessary information from being disclosed, on the basis of the emergency level or the situation of the access to the personal information.

The personal information acquiring section 127 then acquires the accessible personal information from the personal information storing section 18 and supplies it to the notification control section 124, under the control of the personal information access level determining section 126.

The notification control section 124, for example, allows the display section 21 to sequentially display the face image, name, address, age, sex, and blood type of each passenger, the medical information on the passenger, and the passenger's position in the vehicle. The notification control section 124 also controls the voice output section 22 so that information that can be expressed in words can be aurally communicated to the rescuers.

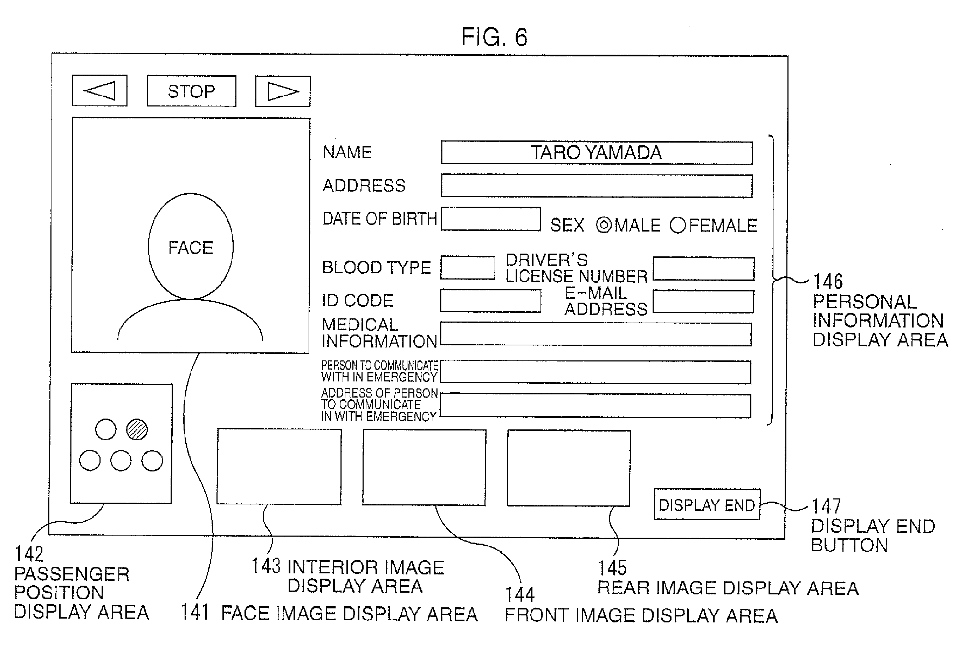

FIG. 6 shows an example of configuration of a personal information display screen.

The personal information display screen displays a face image display area 141, a passenger position display area 142, an interior image display area 143, a front image display area 144, a rear image display area 145, a personal information display area 146, and a display end button 147.

The face image display area 141 displays a face image of a passenger (registered person authenticated to be in the vehicle) acquired, in the registration mode, by the camera provided in the biological information acquiring section 15.

The passenger position display area 142 shows the position of one of the registered persons authenticated to be currently in the vehicle whose face image is being displayed in the face image display area 141. In FIG. 6, the on-vehicle apparatus 1 is mounted in a 5-passenger car. However, if the on-vehicle apparatus 1 is mounted in a passenger car in a different form or a different vehicle, the display of the passenger position display area 142 is differently configured from that in FIG. 6 so as to conform to the vehicle or the like with the on-vehicle apparatus 1.

The front image display area 144 displays an image (either a motion picture or a still image) of external or internal front of the vehicle with the on-vehicle apparatus 1 which image is acquired by the exterior image monitoring section 91 or internal image monitoring section 92 of the emergency detecting section 19 when an emergency is detected.

The rear image display area 145 displays an image (either a motion picture or a still image) of external or internal rear of the vehicle with the on-vehicle apparatus 1 which image is acquired by the exterior image monitoring section 91 or internal image monitoring section 92 of the emergency detecting section 19 when an emergency is detected.

By referencing the image displayed in the front image display area 144 and rear image display area 145, the rescuers can check what the accident causing the emergency is like to deal appropriately with it. The personal information display screen may be able to display information acquired by the emergency detecting section 19 when the emergency is detected. Alternatively, the personal information display screen may be able to display information such as the direction and magnitude of an acceleration generated in the emergency or the speed at which a person's head collides against a surrounding object and which is obtained by analyzing the interior image acquired by the interior image monitoring section 92.

The personal information display area 146 displays the personal information on one of the registered persons authenticated to be currently in the vehicle whose face image is being displayed in the face image display area 141; the personal information has been input, via the operation input section 14, to the personal information input box 73 in the registration screen described with reference to FIG. 3 and are permitted by the personal information access level determining section 126 to be disclosed. The personal information display area 146 displays those pieces of the following information which are permitted to be disclosed: the registered person's name, address, date of birth, sex, blood type, driver's license number, ID code normally required to access the personal information (which may be issued to the user owning the on-vehicle apparatus 1 or which may be set by the user in registering the personal information), and e-mail address, and, for example, medical information such as allergy, contraindication, or medical history which is considered to be critical in an emergency, information on a person to communicate with in an emergency, a contact number or type such as a car insurance, and the organization to which the registered person or the vehicle belongs or its telephone number. The registration in the personal information display area 146 in FIG. 6 is an example and all the information described above may not be provided in the personal information display area 146. Other information, such as whether or not the registered person is registered as a donor can be displayed of course.

The display end button 147 is selected (or depressed) by the user to end the display of the personal information display screen in FIG. 6.

The personal information display screen in FIG. 6 corresponds to the situation described below. The state managing section 122 detects an emergency, and the emergency level determining section 125 determines the level of the emergency. On the basis of the emergency level determined, a face image of a passenger is displayed in the face image display area 141. The apparatus determines that only the name of the passenger be displayed the face image of whom is being displayed in the face image display area 141.

If a plurality of passengers are authenticated to be in the vehicle with the on-vehicle apparatus 1, the face image display area 141 sequentially displays the passengers' face images at intervals of, for example, 3 to 5 seconds. The passenger position display area 142 shows the position of the passenger whose face image is being displayed in the face image display area 141. The personal information display area 146 displays the information permitted to be disclosed of the personal information on the passenger whose face image is being displayed in the face image display area 141.

If a plurality of passengers are authenticated to be in the vehicle with the on-vehicle apparatus 1, the positions of all the passengers are displayed in one screen so that any of the passengers can be chosen to provide a detailed display.

With this configuration, when, for example, an accident occurs and a rescue team arrives at the scene of the accident, a face image of a passenger and required information such as the passenger's name, address, and blood type are displayed on the display section 21 of the on-vehicle apparatus 1. Consequently, even if such a serious accident as causes all the passengers to lose consciousness occurs, the rescuers can deal both promptly and correctly with the situation.

It is assumed that the on-vehicle apparatus 1 is mounted in a public vehicle such as a bus and displays the above personal information in an emergency. Then, when the information is disclosed to a plurality of third persons who are performing rescue operations, it is desirable to allow these persons to access only the required pieces of the information acquired at the time of the passenger getting in the car using electronic tickets, cellular phones, RFIDs, or the like. Further, if the on-vehicle apparatus 1 is mounted in a public vehicle such as a bus and those who got off the bus before the occurrence of the accident may not have been detected, since personal information such as that described above has been registered, an organization such as the police which has a specific authority may utilize registered cellular phone numbers or the like to confirm their whereabouts.

The state managing section 122 determines that the vehicle with the on-vehicle apparatus 1 is likely to have been stolen, upon receiving an input code number which is known only to those concerned with the police or which is registered to allow the check of whether or not the vehicle with the on-vehicle apparatus 1 has been stolen. The response processing section 123 controls the notification control section 124 to notify that a process of checking the owner of the vehicle is to be started. On the basis of a response to the notification, the response processing section 123 confirms that this is not due to an erroneous operation input or a failure in the on-vehicle apparatus 1. The response processing section 123 then notifies the emergency level determining section 125 that the vehicle has been stolen. On the basis of the determination of the emergency level by the emergency level determining section 125, the personal information access level determining section 126 allows the personal information acquiring section 127 to acquire only the face image of a predetermined registered person and then allows the notification control section 124 to display the face image in the face image display area 141. This allows those concerned with the police, third persons, to check whether or not the person in the vehicle with the on-vehicle apparatus 1 is the correct owner or whether or not the vehicle with the on-vehicle apparatus 1 is the one the theft of which has been reported by the correct owner.

Those concerned with the police can reference the face image of the predetermined registered person displayed in the face image display area 141 to check whether or not the person in the vehicle with the on-vehicle apparatus 1 is the correct owner or whether or not the vehicle with the on-vehicle apparatus 1 is the one the theft of which has been reported by the correct owner. Thus, if the vehicle with the on-vehicle apparatus 1 has been stolen, this can be easily determined even if, for example, the number plate or the like has been altered.

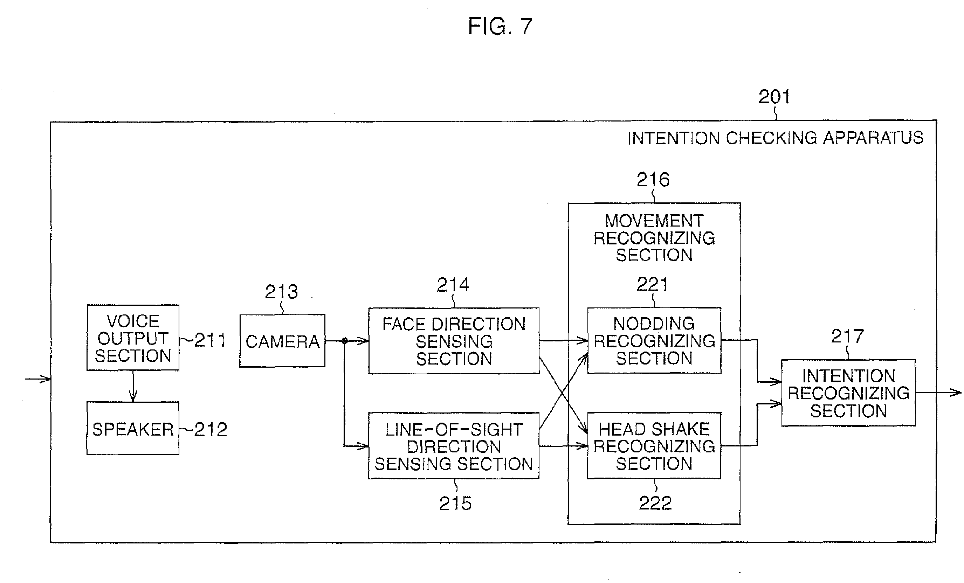

The on-vehicle apparatus 1 is used in, for example, a passenger car as described above. If the on-vehicle apparatus 1 is mounted in a passenger car including an intention checking apparatus 201 described with reference to FIGS. 7 to 17 in order to recognize the passenger's movement indicating his or her intention, it can share some of the functions of the intention checking apparatus 201.

The intention checking apparatus will be described with reference to FIGS. 7 to 17.

FIG. 7 is a block diagram showing an embodiment of the intention checking apparatus 201. The intention checking apparatus 201 includes a voice output section 211, a speaker 212, a camera 213, a face direction sensing section 214, a line-of-sight direction sensing section 215, a movement recognizing section 216, and intention recognizing section 217. The movement recognizing section 216 includes a nodding recognizing section 221 and a head shake recognizing section 222.

If an external application apparatus (not shown; for example, a driving support apparatus, navigation apparatus, car air conditioner, or car stereo) inputs information requesting the check of the driver's intention, to the intention checking apparatus 201, the voice output section 211 generates voice data urging the driver to indicate his or her intention. The voice output section 212 outputs a voice based on the voice data, through the speaker 212. For example, to urge the driver to indicate his or her intention, the voice output section 211 outputs a voice message such as "Settings will be automatically changed. OK?" through the speaker 212.

The camera 213 uses, for example, a CCD (Charge Coupled Device), a CMOS (Complementary Metal Oxide Semiconductor) image pickup element, a logarithmic transformation image pickup element (for example, HDRC (High Dynamic Range CMOS (Complementary Metal Oxide Semiconductor)) (registered trade mark)), or the like. The camera 213 exhibits performance (for example, resolution: 640 (lateral) x 480 (vertical) pixels; the number of gray scales: 256; frame rate: 30 frames per sec) required to sense the directions of the driver's face and the driver's line of sight. The camera 213 is installed at a position where it can pick up an image of the driver's face. The camera 213 supplies image data including the driver's face obtained by image pickup, to the face direction sensing section 214 and the line-of-sight direction sensing section 215.

The face direction sensing section 214 senses the direction of the driver's face on the basis of the image data supplied by the camera 213. The sensing of the face direction by the face direction sensing section 214 is not limited to any particular technique. A technique is desirably used which can sense the face direction both quickly and accurately. The face direction sensing section 214 supplies information indicating the sensed direction of the driver's face, to the nodding recognizing section 221 and the head shake recognizing section 222.

For example, the face direction sensing section 214 senses the face direction based on the state in which the driver looks in a reference direction (for example, a horizontal direction with respect to front of the car and the ground), at an angular resolution of 5° every 0.2 seconds in both vertical and lateral directions. In the description below, for the face direction, the upward direction is indicated by a positive value, while the downward direction is indicated by a negative value on the basis of the state in which the driver looks in the reference direction. The rightward direction is indicated by a positive value, while the leftward direction is indicated by a negative value. For example, if the driver tilts his or her head by 10° upward from the state in which the driver looks in the reference direction, the vertical face direction is 10°. If the driver tilts his or her head by 10° downward, the vertical face direction is -10°. If the driver tilts his or her head by 10° rightward, the lateral face direction is 10°. If the driver tilts his or her head by 10° leftward, the lateral face direction is -10°.

The line-of-sight direction sensing section 215 senses the direction of the driver's line of sight with respect to the driver's face direction on the basis of the image data supplied by the camera 213. The sensing of the line-of-sight direction by the line-of-sight direction sensing section 215 is not limited to any particular technique. A technique is desirably used which can sense the line-of-sight direction both quickly and accurately. The line-of-sight direction sensing section 215 supplies information indicating the sensed direction of the driver's line of sight, to the nodding recognizing section 221 and the head shake recognizing section 222.

For example, the line-of-sight direction sensing 215 senses the driver's line-of-sight direction with respect to the driver's face direction at an angular resolution of 5° every 0.2 seconds in both vertical and lateral directions. In the description below, for the line-of-sight direction, the upward direction is indicated by a positive value, while the downward direction is indicated by a negative value on the basis of the face direction. The rightward direction is indicated by a positive value, while the leftward direction is indicated by a negative value. For example, if the driver moves his or her line of sight by 10° upward with respect to the direction of the driver's face, the line-of-sight direction in the vertical face direction is 10°. If the driver moves his or her line of sight by 10° downward, the line-of-sight direction in the vertical face direction is -10°. If the driver moves his or her line of sight by 10° rightward, the line-of-sight direction in the lateral face direction is 10°. If the driver moves his or her line of sight by 10° leftward, the line-of-sight direction in the lateral face direction is -10°.

The following are not limited to the above example: the time intervals and angular resolution used to sense the face or line-of-sight direction as well as the coordinate system indicating the face or line-of-sight direction.

The movement recognizing section 216 recognizes the driver's movement indicating his or her intention on the basis of the difference between the driver's face direction and line-of-sight direction.

The nodding recognizing section 221 recognizes whether or not the driver has nodded to indicate his or her intention on the basis of the difference between the driver's face direction and line-of-sight direction. The nodding recognizing section 221 supplies information indicative of the recognition to the intention recognizing section 217.

The head shake recognizing section 222 recognizes whether or not the driver has shaken his or her head to indicate his or her intention on the basis of the difference between the driver's face direction and line-of-sight direction.

The head shake recognizing section 222 supplies information indicative of the recognition to the intention recognizing section 217.

The intention recognizing section 217 recognizes the driver's intention on the basis of the recognition supplied by the nodding recognizing section 221 or the head shake recognizing section 222. The intention recognizing section 217 outputs information indicative of the recognition to the application apparatus requesting the check of the driver's intention.

For example, when an external application apparatus (not shown) inputs information requesting the check of the driver's intention, to the on-vehicle apparatus 1, the voice output section 211 urges the driver to indicate his or her intention. Specifically, the voice output section 211 generates voice data urging the driver to indicate his or her intention. The voice output section 211 then causes the speaker 212 to output a voice based on the voice data.

The face direction sensing section 214 starts sensing the face direction on the basis of the image data supplied by the camera 213. The face direction sensing section 214 starts supplying the information indicative of the sensed direction of the driver's face to the nodding recognizing section 221 and the head shake recognizing section 222.

The line-of-sight direction sensing section 215 starts sensing the line-of-sight direction on the basis of the image data supplied by the camera 213. The line-of-sight direction sensing section 215 starts supplying the information indicative of the sensed direction of the driver's face to the nodding recognizing section 221 and the head shake recognizing section 222.

The nodding recognizing section 221 executes a nod recognizing process. The nod recognizing process will be described in detail with reference to FIG. 3. This process recognizes whether or not the driver has nodded in response to a message urging the driver to indicate his or her intention. The nodding recognizing section 221 supplies information indicating the recognition to the intention recognizing section 217.

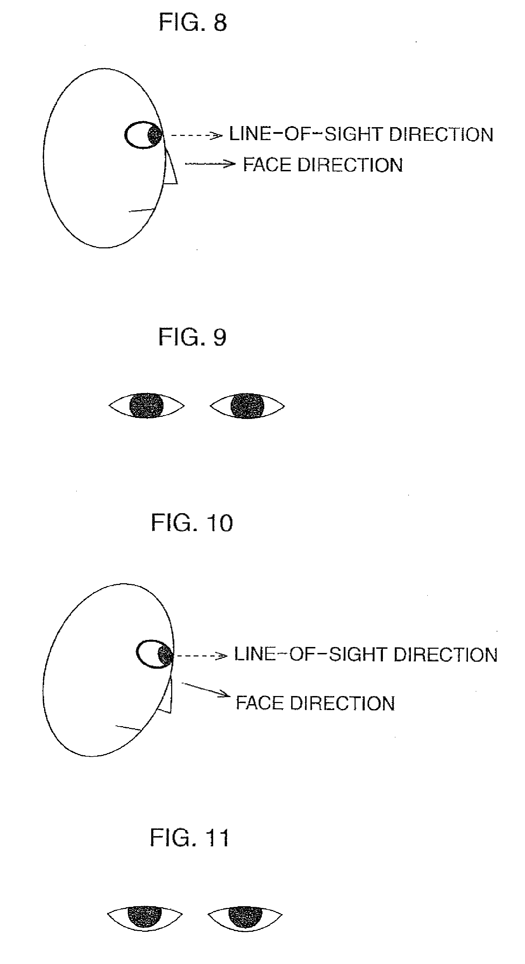

FIG. 8 schematically shows the directions of face and line of sight of a person looking almost straight ahead as viewed from the side of his or her head. FIG. 9 schematically shows a state of the eyes of the person shown in FIG. 8. As shown in FIG. 8, if the person is looking almost straight ahead, for example, if the driver is looking forward while driving a car, the face direction almost coincides with the line-of-sight direction. In spite of the difference among individuals, the iris is located almost in the center of each eye as shown in FIG. 9.

FIG. 10 schematically shows the directions of face and line of sight of a person nodding to indicate his or her intention as viewed from the side of his or her head. FIG. 11 schematically shows a state of the eyes of the person shown in FIG. 10. When a person nods to indicate his or her intention, he or she tends to change only the face direction while almost fixing his or her eyes. For example, while two persons are talking, each of them nods while almost focusing his or her eyes on the other. While performing an operation such as driving, a person nods while almost focusing his or her eyes on the target of the operation. Even if the person has no such a target to gaze, he or she need not look in the direction of the nodding and thus nods while almost fixing his or her eyes.

Accordingly, when a person nods, the face movement deviates from the line-of-sight movement as shown in FIG. 10. That is, the face moves downward, while the line of sight directs upward with respect to the face direction. Thus, in spite of the difference among individuals, the iris moves upward in each eye, that is, in the direction opposite to that of movement of the face as shown in FIG. 11. In other words, the person casts an upward glance.

The nodding recognizing section 221 calculates the likelihood of nodding by noting the difference between the movements (directions) of the face and the line of sight observed when a person nods. Specifically, the likelihood of nodding is calculated in accordance with Equation (1) shown below if the driver's face is located at at least a predetermined first angle (for example, 5°) downward from a reference direction and if the driver's line of sight is located at at least a predetermined second angle (for example, 5°) upward from the face direction and if the leftward or rightward face direction is located at less than a third angle (for example, 5°) from a reference direction, that is, if the driver looks downward and almost in the lateral reference direction, while casting an upward glance.

$$\text{Likelihood of nodding} = \text{vertical line-of-sight direction with respect to the face direction} - \text{vertical face direction} \quad \dots (1)$$

That is, the likelihood of nodding is expressed by the sum of the angle between the face direction and the line-of-sight direction observed when the person casts an upward glance and the angle between the face direction and the line-of-sight direction observed when the person casts a downward glance (Equation (1) is a subtraction because of the signs of the angles).

On the other hand, if the face and line-of-sight directions are not in the above conditions, the likelihood of nodding is zero.

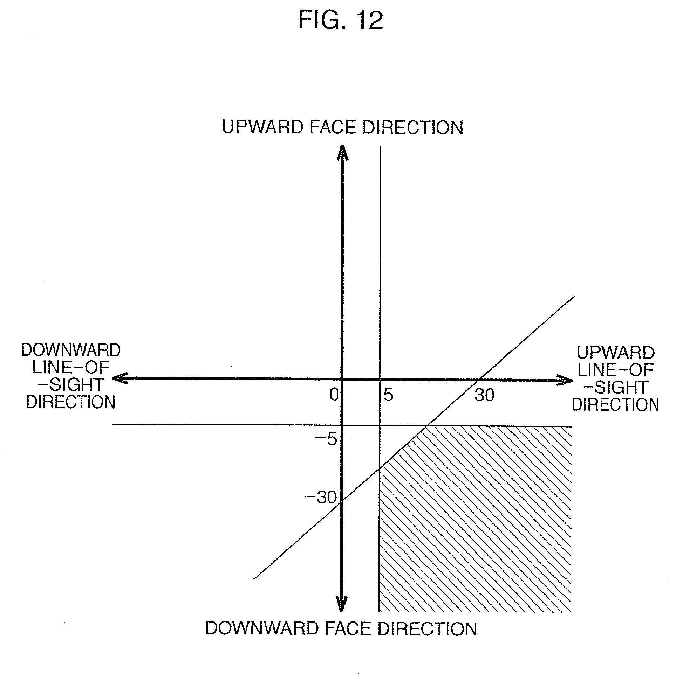

FIG. 12 is a graph in which the axis of abscissa indicates the vertical line-of-sight direction with respect to the face direction, whereas the axis of ordinate indicates the vertical face direction with respect to the reference direction. The angle of the upward line-of-sight direction increases as a point on the axis of abscissa moves rightward from the origin. The angle of the downward line-of-sight direction increases as the point on the axis of abscissa moves leftward from the origin. The angle of the upward face direction increases as a point on the axis of ordinate moves upward from the origin. The angle of the downward face direction increases as the point on the axis of ordinate moves downward from the origin.

In a shaded area in FIG. 12, the likelihood of nodding is at least a predetermined threshold (in the example shown in FIG. 12, 30). That is, in the shaded area, the driver's face is located at at least the predetermined first angle (in the example shown in FIG. 12, 5°) downward from the reference direction, the driver's line of sight is located at at least the predetermined second angle (in the example shown in FIG. 12, 5°) upward from the face direction, and the sum (likelihood of nodding) of the angle between the downward face direction and the reference direction and the angle between the upward line-of-sight direction and the face direction is at least the predetermined threshold (in the example shown in FIG. 12, 30).

The nodding recognizing section 221 calculates the likelihood of "nodding" on the basis of such a method as described above and supplies information indicative of the recognition to the intention recognizing section 217.

The head shake recognizing section 222 executes a head shake recognizing process.

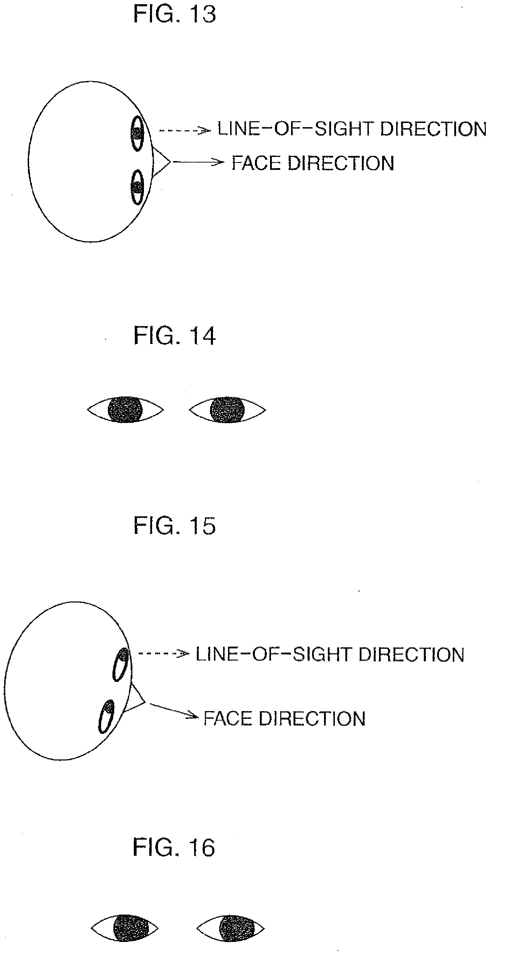

FIG. 13 schematically shows the directions of face and line of sight of a person looking almost straight ahead as viewed from above his or her head. FIG. 14 schematically shows a state of the eyes of the person shown in FIG. 13. As shown in FIG. 13, if the person is looking almost straight ahead, for example, if the driver is looking forward while driving a car, the face direction almost coincides with the line-of-sight direction. In spite of the difference among individuals, the iris is located almost in the center of each eye as shown in FIG. 14.

FIG. 15 schematically shows the directions of face and line of sight of a person shaking his or her head to indicate his or her intention as viewed from above his or her head. FIG. 16 schematically shows a state of the eyes of the person shown in FIG. 15. When a person shakes his or her head to indicate his or her intention, he or she tends to change only the face direction while almost fixing his or her eyes. For example, while two persons are talking, each of them shakes his or her head while almost focusing his or her eyes on the other. While performing an operation such as driving, a person shakes his or her head while almost focusing his or her eyes on the target of the operation. Even if the person has no such a target to gaze, he or she need not look in the direction of the head shaking and thus shakes his or her head while almost fixing his or her eyes.

Accordingly, when a person shakes his or her head, the face direction deviates from the line-of-sight direction as shown in FIG. 15. That is, the face direction is opposite to the line-of-sight direction. Thus, in spite of the difference among individuals, the iris moves, in each eye, in the direction (in this case, leftward) opposite to that of movement of the face (in this case, rightward) as shown in FIG. 16. In other words, the person casts a side glance.

The head shake recognizing section 222 calculates the likelihood of head shaking by noting the difference between the movements (directions) of the face and the line of sight observed when a person shakes his or her head. Specifically, the likelihood of head shaking is calculated in accordance with Equation (2) shown below if the driver's face is located at at least a predetermined first angle (for example, 5°) rightward or leftward from a reference direction and if the driver's line of sight is located at at least a predetermined second angle (for example, 5°) from the lateral face direction in the direction opposite to the face direction and if the upward or downward face direction is located at less than a third angle (for example, 5°) from a reference direction, that is, if the driver looks leftward or rightward and almost in the vertical reference direction, while casting a glance in the direction laterally opposite to the face direction.

Likelihood of head shaking = lateral line-of-sight direction with respect to the face direction - lateral face direction ... (2)

That is, the likelihood of head shaking is expressed by the sum of the angle between the face direction and the line-of-sight direction observed when the person casts an side glance and the angle between the face direction and the line-of-sight direction observed when the person's head is directed sideward (Equation (2) is a subtraction because of the signs of the angles).

On the other hand, if the face and line-of-sight directions are not in the above conditions, the likelihood of head shaking is zero.

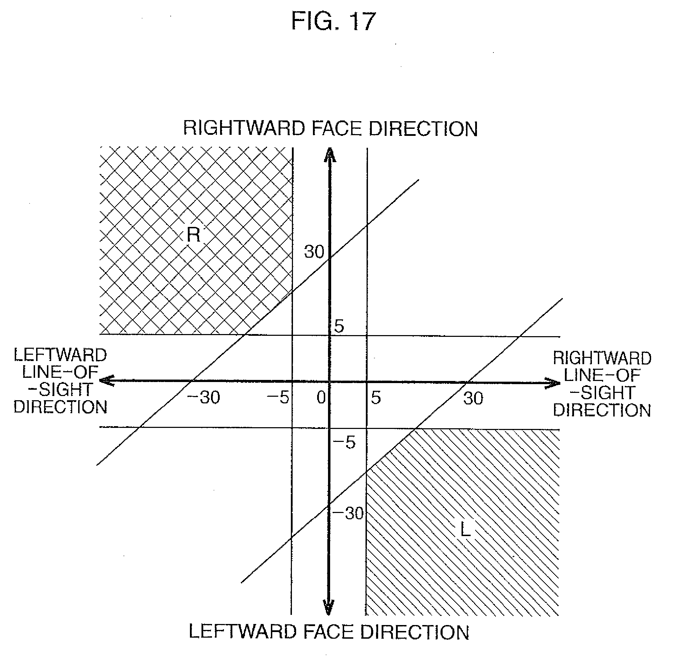

FIG. 17 is a graph in which the axis of abscissa indicates the lateral line-of-sight direction with respect to the face direction, whereas the axis of ordinate indicates the lateral face direction with respect to the reference direction. The angle of the rightward line-of-sight direction increases as a point on the axis of abscissa moves rightward from the origin. The angle of the leftward line-of-sight direction increases as the point on the axis of abscissa moves leftward from the origin. The angle of the rightward face direction increases as a point on the axis of ordinate moves upward from the origin. The angle of the leftward face direction increases as the point on the axis of ordinate moves downward from the origin.

In a meshed area R in FIG. 17, the likelihood of head shaking is an area below a predetermined lower-limit threshold when the face is directed rightward (in the example shown in FIG. 17, -30). That is, in the area R, the driver's face is located at at least the predetermined first angle (in the example shown in FIG. 17, 5°) rightward from the reference direction, the driver's line of sight is located at at least the predetermined second angle (in the example shown in FIG. 17, 5°) leftward from the face direction, and the sum (likelihood of head shaking) of the angle between the rightward face direction and the reference direction and the angle between the leftward line-of-sight direction and the face direction is at least the predetermined threshold (in the example shown in FIG. 17, 30 (absolute value of the lower-limit threshold)).

In a shaded area L in FIG. 17, the likelihood of head shaking is an area above a predetermined upper-limit threshold when the face is directed leftward (in the example shown in FIG. 17, 30). That is, in the area L, the driver's face is located at at least the predetermined first angle (in the example shown in FIG. 17, 5°) leftward from the reference direction, the driver's line of sight is located at at least the predetermined second angle (in the example shown in FIG-17, 5°) rightward from the face direction, and the sum (likelihood of head shaking) of the angle between the leftward face direction and the reference direction and the angle between the rightward line-of-sight direction and the face direction is at least the predetermined threshold (in the example shown in FIG. 17, 30).

The head shake recognizing section 222 calculates the likelihood of "head shaking" on the basis of the above method.

The intention recognizing section 217 recognizes the driver's intention on the basis of the recognition of the nodding recognizing section 221 or the head shake recognizing section 222 to finish the intention recognizing process. Specifically, if the nodding recognizing section 221 recognizes that the driver has nodded, while the head shake recognizing section 222 recognizes that the driver has not shaken his or her head, the intention recognizing section 217 recognizes that the driver has indicated affirmation. If the nodding recognizing section 221 recognizes that the driver has not nodded, while the head shake recognizing section 222 recognizes that the driver has shaken his or her head, the intention recognizing section 217 recognizes that the driver has indicated negation. Further, if the nodding recognizing section 221 recognizes that the driver has not nodded and the head shake recognizing section 222 recognizes that the driver has not shaken his or her head, the intention recognizing section 217 recognizes that the driver has not indicated his or her intention.

If the nodding recognizing section 221 recognizes that the driver has nodded and the head shake recognizing section 222 recognizes that the driver has shaken his or her head, the intention recognizing section 217 recognizes the driver's intention on the basis of his or her movement recognized earlier or later. Alternatively, the driver's intention may be recognized on the basis of the likelihood of nodding on the basis of which whether or not the driver has nodded is determined and the likelihood of head shaking on the basis of which whether or not the driver has shaken his or her head is determined.

The intention recognizing section 217 outputs information indicating the recognition to the application apparatus requesting the check of the driver's intention. The application apparatus executes a process based on the recognized intention of the driver.

If the on-vehicle apparatus 1 is mounted in, for example, a passenger car together with the intention checking apparatus 201, described with reference to FIGS. 7 to 17, it may share some of the functions of the intention checking apparatus 201 (for example, the speaker 212, the camera 213, and the image recognizing function of processing and recognizing an image acquired via the camera 213).

If the on-vehicle apparatus 1 is mounted in, for example, a passenger car together with the intention checking apparatus 201, described with reference to FIGS. 7 to 17, the driver's face image is constantly acquired and can thus be used to execute an authenticating process as required.

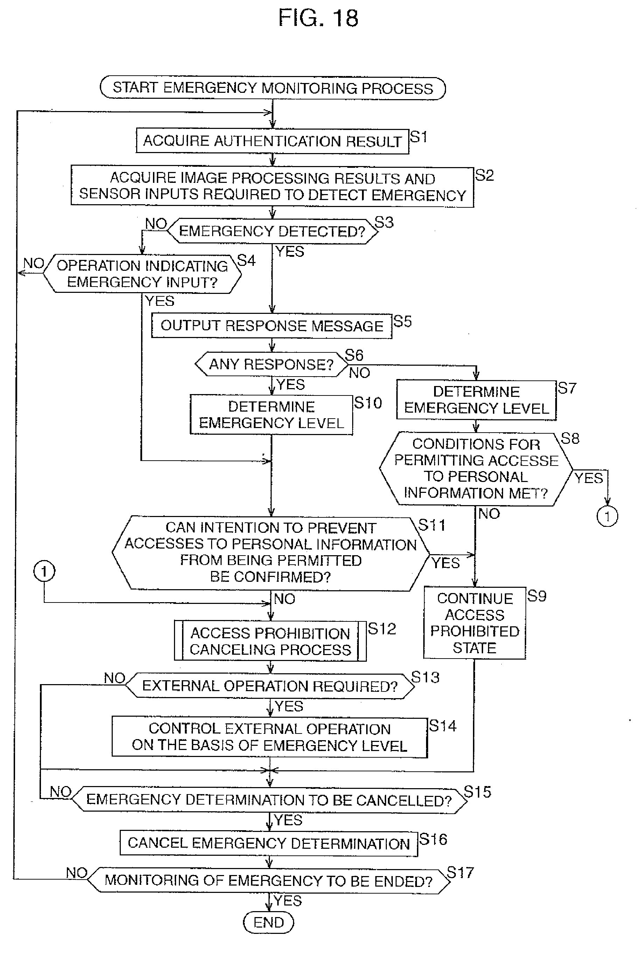

Now, an emergency monitoring process executed by the on-vehicle apparatus 1 will be described with reference to the flowchart in FIG. 18.

In step S1, the authentication result acquiring section 121 acquires an authentication result provided by the authentication processing section 16 and stored in the authentication result storing section 17. The authentication result acquiring section 121 then supplies the result to the state managing section 122. The notification control section 124 also acquires the authentication result provided by the authentication processing section 16.

In step S2, the state managing section 122 acquires image processing results and sensor inputs required to detect an emergency, from the emergency detecting section 19.

In step S3, the state managing section 122 determines whether or not an emergency has been detected, on the basis of the image processing results and sensor inputs supplied by the emergency detecting section 19.

If the state managing section 122 determines in step S3 that no emergency has been detected, then it determines in step S4 whether or not an operation indicative of an emergency has been input, for example, whether or not there has been an input of a code number known only to those concerned with the police or registered to allow the check of whether or not the vehicle or the like with the on-vehicle apparatus 1 has been stolen.

If the state managing section 122 determines in step S4 that an operation indicative of an emergency has been input, the process proceeds to step S11 described later. If the state managing section 122 determines in step S4 that no operation indicative of an emergency has been input, the process returns to step S1 to repeat the subsequent steps.

If the state managing section 122 determines in step S3 that an emergency has been detected, then in step S5, it causes the response processing section 123 to provide a message checking the passenger's intention. The response processing section 123 controls the notification control section 124 to output an aural or visual response message to the user in order to determine the level of the emergency.

In step S6, the response processing section 123 determines whether or not the user has responded to the message, on the basis of the user's operation input supplied by the operation input section 14. If the response processing section 123 determines in step S 6 that the user has responded to the message, the process proceeds to step S10 described later.

If the response processing section 123 determines in step S6 that the user has not responded to the message, then in step S7, it notifies the emergency level determining section 125 that the user has not responded. The emergency level determining section 125 determines the emergency level on the basis of the state of the vehicle with the on-vehicle apparatus 1, managed by the state managing section 122, and the lack of the user's response. The emergency level determining section 125 then supplies the emergency level to the personal information access level determining section 126.

Specifically, the state managing section 122 receives an input signal indicative of emergency, for example, a collision, turning of the vehicle, or a sudden, high acceleration generated in a direction different from the advancing direction. Then, if the user has not responded to the message, the emergency level determining section 125 determines that the emergency is at a high level.

In step S8, the personal information access level determining section 126 determines whether or not the emergency level, a person accessing the personal information, or the type of the vehicle with the on-vehicle apparatus 1, or the like meets the corresponding condition for permitting accesses to the personal information, as described above. If the personal information access level determining section 126 determines in step S8 that the condition for permitting accesses to the personal information is met, the process proceeds to step S12 described later.

If the personal information access level determining section 126 determines in step S8 that the condition for permitting accesses to the personal information is not met, then in step S9, it continuously prohibits accesses to continuously inhibit the personal information from being disclosed. The process proceeds to step S15 described later.

If the response processing section 123 determines in step S6 that the user has responded to the message, then in step S10, it notifies the emergency level determining section 125 of the contents of the user's response. The emergency level determining section 125 determines the emergency level on the basis of the state of the vehicle with the on-vehicle apparatus 1, managed by the state managing section 122, and the contents of the user's response. The emergency level determining section 125 then supplies the emergency level to the personal information access level determining section 126.

In step S11, the personal information access level determining section 126 determines whether or not it is possible to confirm the intention of the user of the on-vehicle apparatus 1 to continuously prohibit accesses to the personal information on the basis of the contents of the user' s response supplied. If the personal information access level determining section 126 determines in step S11 that the intention to continuously prohibit accesses to the personal information has been confirmed, the process proceeds to step S9 described above.

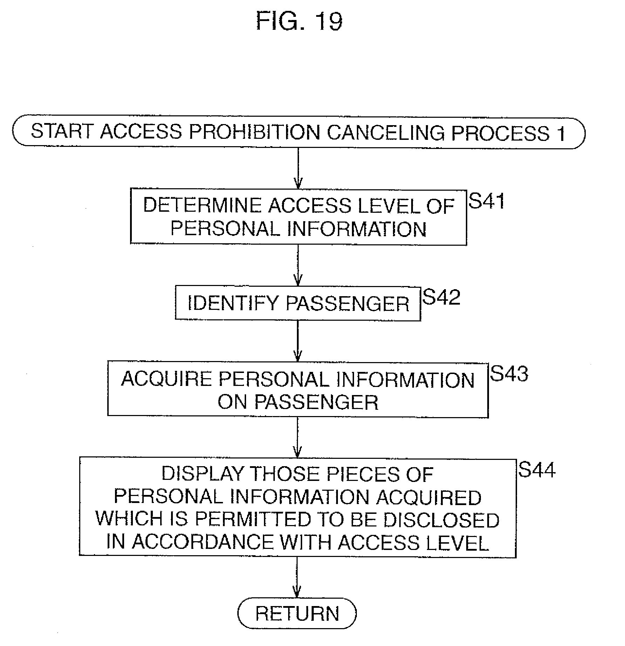

If the personal information access level determining section 126 determines in step S8 that the condition for permitting accesses to the personal information is met or if the personal information access level determining section 126 determines in step S11 that the intention to continuously prohibit accesses to the personal information has not been confirmed, then an access prohibition canceling process described later with reference to FIG. 19 is executed.

In step S13, the emergency level determining section 125 determines whether or not an external operation is required, on the basis of the determination of the emergency level.

If the emergency level determining section 125 determines that an external operation is required in step S13, then in step S14, it supplies the determination of the emergency level to the external operation control section 128. The external operation control section 128 controls the external operation section 20 on the basis of the emergency level to cause it to perform an external operation, for example, of unlocking the key to the vehicle or automatically opening the doors, so that the passengers can promptly get out of the car to take refuge or the rescuers can promptly check the interior of the vehicle to rescue the passengers as required.

If the emergency level determining section 125 determines after the processing in step S9 or during step S13 that no external operation is required, or after the processing in step S14 is finished, then the state managing section 122 determines in step S15 whether or not the emergency determination is to be cancelled, for example, on the basis of the contents of the operation input in the operation input section 14. If the state managing section 122 determines in step S15 that the emergency determination not be cancelled, the processing in step S15 is repeated until the state managing section 122 determines that the emergency determination be cancelled.

If the state managing section 122 determines in step S15 that the emergency determination be cancelled, then in step S16, the state managing section 122 cancels the emergency determination.

In step S17, the state managing section 122 determines whether or not the monitoring of an emergency is to be ended, for example, on the basis of the contents of the operation input in the operation input section 14. If the state managing section 122 determines in step S17 that the monitoring of an emergency not be ended, the process returns to step S1 to repeat the subsequent steps. If the state managing section 122 determines in step S17 that the monitoring of an emergency be ended, the process is finished.

This process monitors an emergency, and if an emergency occurs, determines the level of emergency. The process then determines whether or not to cancel access prohibition on the basis of the determination of the emergency level.

This enables persons such as rescuers who deal with the emergency to reference information required to perform required operations on the scene, together with face images without the need for special equipment. The rescuers can thus deal both appropriately and promptly with the situation. The process also prevents the leakage of more secret information than required.

Now, with reference to the flowchart in FIG. 19, description will be given of an access prohibition canceling process 1 executed in step S12 in FIG. 18.

In step S41, the personal information access level determining section 126 determines the access level of the personal information on the basis of the determination of the emergency level made by the emergency level determining section 125 and on the basis of the operation input by a person requesting an access to the personal information and supplied by the operation input section 14, as required.

In step S42, the personal information access level determining section 126 identifies each passenger on the basis of information supplied by the emergency level determining section 125.

In step S43, the personal information access level determining section 126 controls the acquisition of the personal information by the personal information acquiring section 127 on the basis of the determination of access level of the personal information and the information on the passenger. Under the control of the personal information access level determining section 126, the personal information acquiring section 127 acquires the personal information on the passenger permitted to be accessed and supplies it to the notification control section 124.

In step S44, the notification control section 124 displays, on the basis of the authentication result for the passenger, those pieces of the personal information supplied by the personal information acquiring section 127 which are permitted to be disclosed in accordance with the access level. Specifically, the notification control section 124 allows the display section 21 to sequentially display the passenger's faceimage, name, address, age, sex, and blood type, the medical information on the passenger, and the passenger's position in the vehicle. The notification control section 124 also controls the voice output section 22 so that information that can be expressed in words can be aurally communicated to the rescuers. After step S44 is ended, the process returns to step S12 in FIG. 18 and then proceeds to step S13.

If such a process has cancelled the access prohibition, the personal information access level determining section 126 determines the access level of the personal information. For the person identified as a passenger, only the personal information suitable for the emergency is disclosed and not unnecessary personal information.

Since an emergency vehicle or the like may not be able to immediately approach the target, the personal information can be suitably remotely accessed via a network. A communication function with external apparatuses may thus be mounted in the on-vehicle apparatus to notify an external apparatus of the detected contents of the emergency and the detected personal information.

Moreover, when the personal information can be registered in a server or the like, even if a person not registered in the on-vehicle apparatus is in the vehicle, the personal information on that passenger can be retrieved from the information registered in the server. The personal information on this passenger can thus be displayed or communicated to an external apparatus.

Further, if the on-vehicle apparatus can communicate wi th a server managed by an insurance company or a car dealer, then in an emergency, the required personal information and the contents of the emergency such as an accident can be quickly communicated. This makes it possible to promptly execute processes required after the accident.

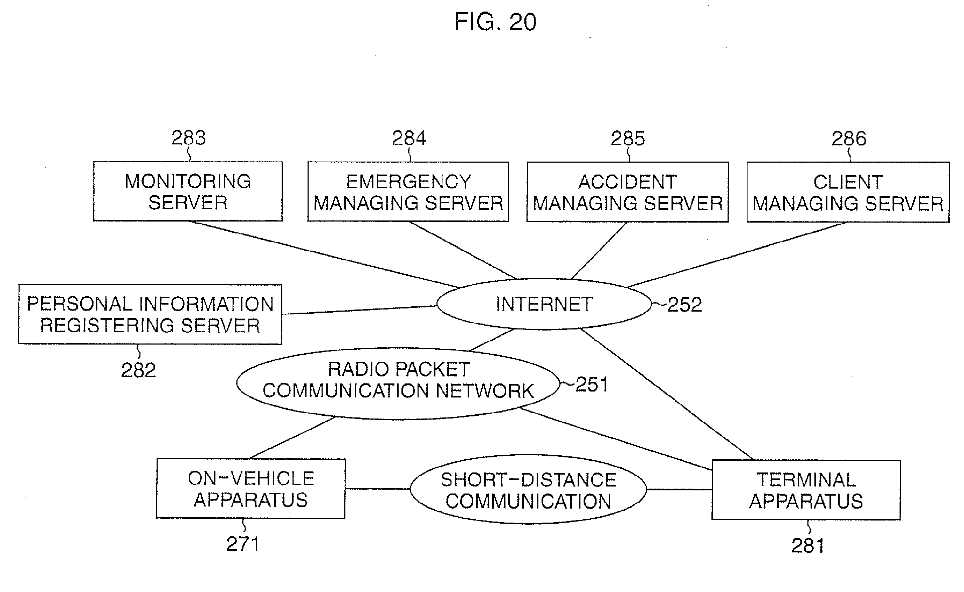

With reference to FIG. 20, description will be given of an emergency personal information providing system to which the present invention is applied.

An on-vehicle apparatus 271 to which the present invention is applied is connected to a wide area network such as the Internet 252 via a radio packet communication network 251. The on-vehicle apparatus 271 can thus transmit and receive information to and from a personal information registering server 282, a monitoring server 283, an emergency managing server 284, an accident managing server 285, and a client managing server 286.

If the on-vehicle apparatus 271 has, for example, a short-distance communication function that uses Bluetooth (TM), it can communicate with a terminal apparatus 281 also having ashort-distancecommunicationfunction. The terminal apparatus 281 may be a cellular phone, small-sized personal computer, or PDA which comprises, for example, a Bluetooth short-distance communication function, or a dedicated terminal. The terminal apparatus 281 may also be configured as a part of the on-vehicle apparatus 271. The terminal apparatus 281 can also be connected to the personal information registering server 282, monitoring server 283, emergency managing server 284, accident managing server 285, and client managing server 286 via the Internet 252 or via the radio packet communication network 251 and Internet 252.

If a person having the terminal apparatus 281 passes by a vehicle involved in an accident or the like or rushes to the scene of the accident for a rescue operation, he or she can promptly perform the rescue operation provided that he or she can receive the contents of the accident or the personal information on the passengers from the on-vehicle apparatus 271. If the terminal apparatus 281 is held by a particular person such as a rescue team member or a police officer, a relatively wide range of personal information on the passengers may be transmitted by the on-vehicle apparatus. However, if the terminal apparatus 281 is held by a general person, the minimum required information on the passengers is preferably transmitted by the terminal apparatus 271 in view of privacy. If the terminal apparatus 281 is held by both the particular person such as a rescue team member or a police officer and the general person, the personal information on the passengers transmitted by the on-vehicle apparatus suitably varies depending on the holder of the receiving terminal apparatus 281.

The personal information registering server 282 is managed by, for example, a company providing network services that use the on-vehicle apparatus 271. The personal information registered in the on-vehicle apparatus 271 is supplied to and registered in the client managing server 286 via the radio packet communication network 251 and the Internet 252.

Thus, if, for example, a passenger not registered in the on-vehicle apparatus 271 is in the vehicle with the on-vehicle apparatus 271, the personal information on that passenger can be acquired and disclosed by inquiring of the personal information registering server 282. Even if the personal information registered in the on-vehicle apparatus 271 cannot be read in an accident, the personal information on the passengers can be acquired and disclosed by inquiring of the personal information registering server 282 for the recorded results of authentication of the passengers executed in the on-vehicle apparatus 271.

The monitoring server 283 is managed by a security company or a company or organization that provides mayday services. Upon receiving the contents of the accident or the personal information on the passengers from the on-vehicle apparatus 271 via the radio packet communication network 251 and the Internet 252, the monitoring server 283 checks the contents of the emergency and makes a report corresponding to the contents of the emergency. For example, upon receiving a notification requesting only a report of the accident, from a passenger in the vehicle with the on-vehicle apparatus 271 after a slight accident occurs, the monitoring server 283 reports the passengers' names and the location of the scene of the accident to the police (or accident managing server 285). Upon receiving a notification indicating that a serious accident is likely to have occurred to damage the user, who has been confirmed not to respond to the message, the monitoring server 283 communicates the detected contents of the emergency and the detailed personal information on the passengers to the emergency managing server 284 in a firehouse, the accident managing server 285 in a police station, and the client managing server 286 in the insurance company or car dealer via the internet 252.

The emergency managing server 284 is managed by, for example, an organization such as a firehouse or a private emergency vehicle providing company which manages the dispatch of emergency vehicles or a medical institution that accepts emergency patients. The emergency managing server 284 receives information on the contents of the accident or the location of the scene of the accident and the required personal information from the monitoring server 283 or the on-vehicle apparatus 271. The emergency managing server 284 then dispatches emergency vehicles to the scene of the accident as required.

The accident managing server 285 is managed by, for example, by the police. The accident managing server 285 receives the information on the contents of the accident or the location of the scene of the accident and the required personal information from the monitoring server 283 or the on-vehicle apparatus 271. The accident managing server 285 then dispatches emergency vehicles to the scene of the accident as required.

The client managing server 286 is managed by, for example, a security company, an insurance company, or a car dealer. The client managing server 286 receives the information on the contents of the accident or the location of the scene of the accident and the required personal information from the monitoring server 283 or the on-vehicle apparatus 271. The client managing server 286 then starts services based on a security contract or an insurance contract.

For example, if the client managing server 286 is managed by an insurance company and the on-vehicle apparatus 271 detects an emergency, the insurance company automatically starts their services. Further, the insurance company and the contractor can predetermine whether or not to disclose the individual information depending on the emergency level or can contract to set the insurance rate depending on the contents of the disclosed personal information.

This configuration allows the contractor to reliably communicate information required to implement the contract, to the insurance company, while eliminating the need to disclose, to the insurance company, information which is unrelated to the insurance services and which is desirably unknown to the others. It is also possible to avoid preventing the application of the insurance as a result of a failure to prove the truth. Further, the insurance company can set detailed insurance rates enough to deal with possible risks, thus reducing the risks. When an accident or the like occurs, the user cannot make a false report and the contents of the emergency can be determined without the need to devote much time and effort to investigations as in the prior art. This enables compensations based on the truth. Moreover, during various investigating processes, the insurance services can be appropriately provided without acquiring information that the contractor desires to keep a secret. This is expected to prevent clients from hesitating to enter into an insurance contract by being afraid that they must disclose the personal information against his or her will before they can receive services.

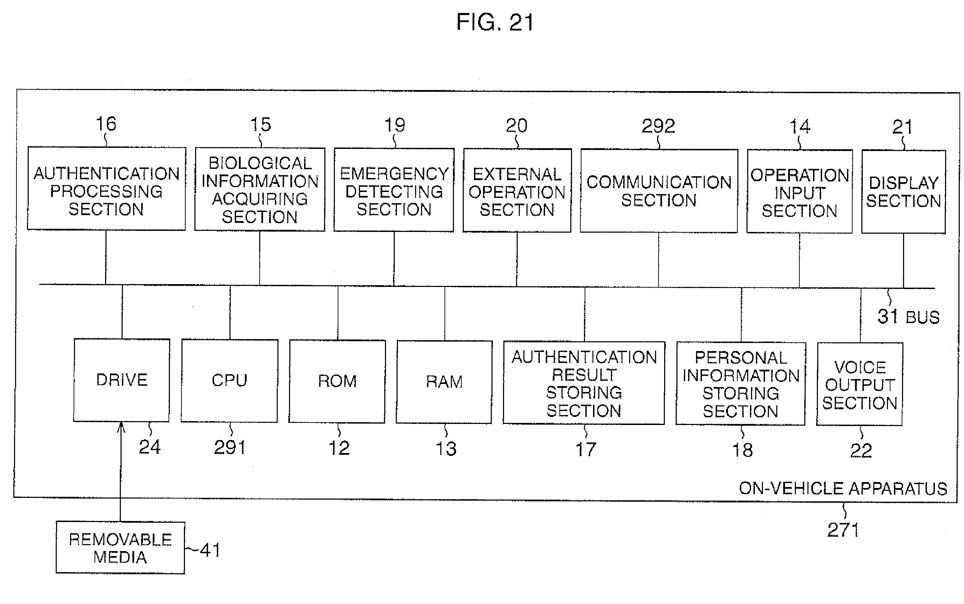

FIG. 21 is a block diagram showing the configuration of the on-vehicle apparatus 271. In this figure, the same components as those of the on-vehicle apparatus 1, described with reference to FIG. 1, are denoted by the same reference numerals. Their description will be omitted as required.

The on-vehicle apparatus 271 is configured similarly to the on-vehicle apparatus 1, described with reference to FIG. 1, except that a CPU 291 is provided in place of the CPU 11 and that a communication section 292 is provided in place of the communication section 23.

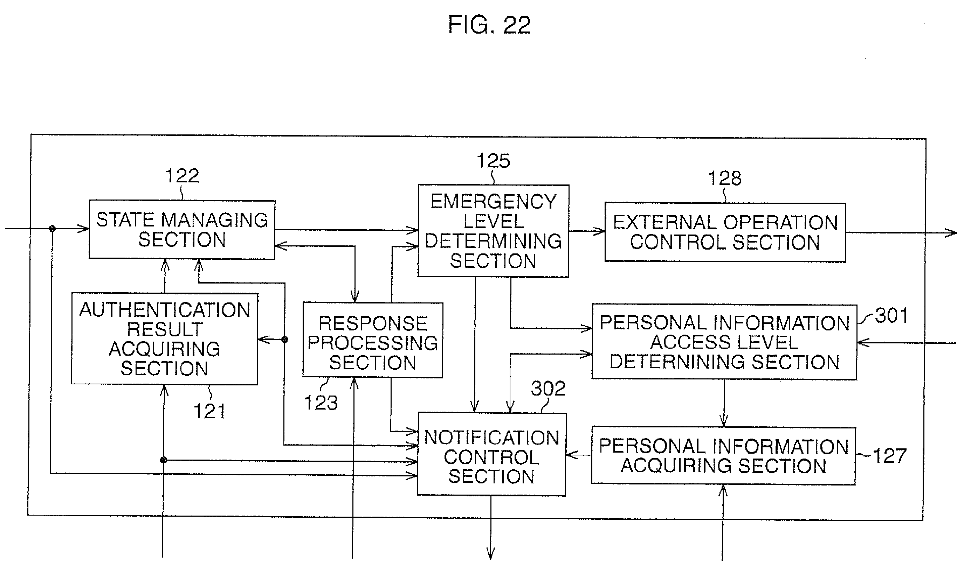

The CPU 291 controls the operation of each section of the on-vehicle apparatus 271. With reference to FIG. 22, a detailed description will be given of functions that can be provided by the CPU 291 by executing predetermined application programs stored in the ROM 12.

The communication section 292 is connected to a wide area network, for example, the Internet 252, or the radio packet communication network 251 by wire or radio to transmit and receive information to and from external apparatuses. The communication section 292 can communicate with the personal information registering server 282, monitoring server 283, emergency managing server 284, accident managing server 285, and client managing server 286 via the Internet 252 or via the radio packet communication network 251 and Internet 252 by, for example, packet communication or short-distance communication using Bluetooth (TM).

FIG. 22 is a functional block diagram illustrating the functions provided by the CPU 291 by executing the predetermined application programs. In this figure, the same components as those in the functional block diagram in FIG. 5 are denoted by the same reference numerals. Their description will be omitted as required.

The CPU 291 has functions basically similar to those which can be provided by the CPU 11, described with reference to FIG. 5, except that a personal information access level determining section 301 is provided in place of the personal information access level determining section 126 and that a notification control section 302 is provided in place of the notification control section 124.

The functions (function modules) provided by the CPU 291 by executing the predetermined programs include the authentication result acquiring section 121, the state managing section 122, the response checking section 123, the notification control section 302, the emergency level determining section 125, the personal information access level determining section 301, the personal information acquiring section 127, and the external operation control section 128.

The personal information access level determining section 301 determines the access level of the personal information on the basis of the determination of the emergency level by the emergency level determining section 125 and the operation input by a person requesting an access to the personal information which input is supplied by the operation input section 14, and as required, on the basis of the destination of the personal information if the information is transmitted by short-distance radio communication or packet communication. The personal information access level determining section 301 thus controls the acquisition of the personal information by the personal information acquiring section 127.

Under the control of the response checking section 123, the notification control section 302 communicates an aural or visual message to the user in order to determine the emergency level. The communication control section 302 controls the display section 21 or voice output section 22 to cause it to display the personal information on each authenticated passenger (who is in the vehicle with the on-vehicle apparatus 1) in accordance with the determination of the emergency level and the determination of the personal information access level under the control of the emergency level determining section 125. The communication control section 302 also controls the communication section 292 to cause it to transmit the message to an external apparatus.

Now, description will be given of an example of detection of an emergency and operations performed after the detection.

If the emergency detecting section 19 detects an emergency, for example, a collision, turning of the vehicle, or a sudden, high acceleration generated in a direction different from the advancing direction, the state managing section 122 determines the possibility of a collision to allow the response processing section 123 to provide a message asking the passengers' intention.

The response processing section 123 controls the notification control section 302 to cause it to give an aural or visual message to the users in order to determine the level of the emergency. The notification control section 302 controls the display section 21 or the voice output section 22 to cause it to output a visual or aural message asking the users' intention, for example, "Are you all right? Would you mind notifying the monitor center (or the police, a firehouse, or surrounding people) of the emergency?". If the emergency is critical, for example, the leakage of fuel is detected, the response processing section 123 suitably controls the notification control section 302 to cause it to output a message notifying the users of a danger, for example, "Fuel is leaking. A fire may break out. Stop the engine and get out of the car."

The response processing section 123 notifies the emergency level determining section 125 whether or not any of the passengers has responded.

The emergency level determining section 125 determines the emergency level on the basis of the result of detection of the emergency supplied by the state managing section 122 as well as whether or not any of the passengers has responded. If the emergency level determining section 125 determines that the emergency level is high, the external operation control section 128 controls the external operation section 20 to cause it to, for example, unlock the key to the vehicle or automatically open the doors, so that the passengers can promptly get out of the car to take refuge or the rescuers can promptly check the interior of the vehicle to rescue the passengers as required.

The personal information access level determining section 301 determines the level of the disclosed personal information on the basis of the determination of the emergency level by the emergency level determining section 125 and on the basis of the operation input by a person requesting an access to the personal information which input is supplied by the operation input section 14.

Like the personal information access level determining section 126, the personal information access level determining section 301 must be able to select the disclosed information in accordance with conditions such as the emergency level, the identification of the person requesting an access to the personal information (or checking the displayed personal information), the disclosed group set at the time of registration, and the type of the vehicle with the on-vehicle apparatus 1. Further, if the personal information needs to be transmitted to an external apparatus via short-distance radio communication or the radio packet communication network 251 and Internet 252, the personal information access level determining section 301 must be able to select the type of the disclosed personal information in accordance with conditions for the receiver of the personal information.

The personal information acquiring section 127 then acquires the accessible personal information from the personal information storing section 18 and supplies it to the notification control section 302, under the control of the personal information access level determining section 301.

Similarly to the notification control section 124 of the on-vehicle apparatus 1, described above, the notification control section 302, for example, allows the display section 21 to sequentially display the face image, name, address, age, sex, and blood type of each passenger, the medical information on the passenger, and the passenger's position in the vehicle, and controls the voice output section 22 to cause it to give an aural message to the passenger. The notification control section 302 further transmits the personal information and the information on the emergency to the rescuers' terminal apparatus 281 by short-distance communication, for example, Bluetooth (TM) or transmits the personal information and the information on the emergency to the monitoring server 283, emergency managing server 284, accident managing server 285, and client managing server 286 via the radio packet communication network 251 and Internet 252 as required.

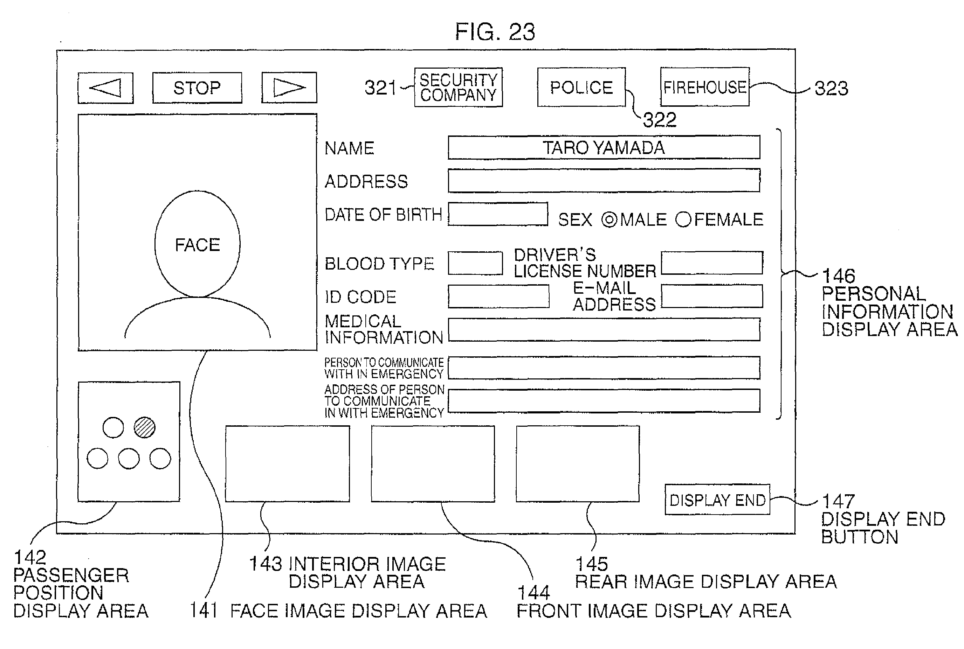

FIG. 23 shows an example of configuration of a personal information display screen. In this figure, the same components as those of the personal information display screen described with reference to FIG. 6 are denoted by the same reference numerals. Their description will be omitted as required.

The personal information display screen in FIG. 23 is configured basically similarly to that described with reference to FIG. 6 except that a security company notifying button 321, a police reporting button 322, and a firehouse reporting button 323 are newly provided.

If, for example, a rescuer such as a rescue team member selects (depresses) the security company notifying button 321 on the personal information display screen, the client managing server 286 is automatically notified of the emergency. This facilitates processes such as communications with the family members with which, for example, the security company, insurance company, or car dealer is pre-entrusted.

If, for example, the rescue team member determines on the basis of the situation on the scene that the emergency is not an accident but a case of bodily injury and selects (depresses) the police reporting button 322 on the personal information display screen, the police (accident managing server 287) receive information on the passengers, a motion picture of the interior of the vehicle taken immediately before the occurrence of the emergency, and face images of and personal information on the passengers or likely passengers. The police can thus acquire detailed information on the case to immediately deal with it.

If a member of the public who is not a rescue team member and the like approaches the scene of the accident for a rescue operation, he or she may be frightened and fail to take an appropriate action. In such a case, the security company notifying button 321, police reporting button 322, and firehouse reporting button 323 enable appropriate information to be quickly communicated to an appropriate destination. If a member of the public who is not a rescue team member and the like approaches the scene of the accident for a rescue operation, the personal information is desirably prevented from being disclosed to that person as in the case of the on-vehicle apparatus 1, described above. Thus, for example, the personal information display screen may display only the minimum required personal information, while detailed personal information such as a clinical history may be transmitted to the emergency managing server 284; the detailed personal information is desirably prevented from being widely disclosed but is required for emergency medical treatment.

In the above description, if the security company notifying button 321, police reporting button 322, or firehouse reporting button 323 is selected or depressed, the information is transmitted to the destination by communication via the network. However, instead of communicating the information via the network, a telephone call may be given to the security company, police, firehouse, or the like by selecting or depressing the security company notifying button 321, police reporting button 322, or firehouse reporting button 323. In this case, what is called video telephone function can be suitably utilized.

The notification control section 302 of the on-vehicle apparatus 271 controls the communication section 292 so that the personal information on the passengers and the information on the emergency can be communicated to the rescuers' terminal apparatus 281 by short-distance communication such as Bluetooth (TM). Thus, if, for example, an emergency vehicle rushing to the scene of the accident comes within a short-distance communication range, it can receive the personal information on the passengers and the information on the emergency transmitted by the communication section 292. The rescue team can thus predetermine how to deal with the situation. In particular, the conditions described below enable the rescuers to determine the situation of the damage in much detail before the emergency vehicle reaches the scene of the accident. The terminal apparatus 281 has a function of displaying images. The terminal apparatus 281 receives image information on the accident acquired at the time of occurrence and which is similar to that displayed in the interior image display area 143, front image display area 144, and rear image display area 145 of the personal information display screen described with reference to FIG. 23 so that the rescuers can reference the information. The terminal apparatus 281 can notify the rescuers of the acceleration of the vehicle measured at the time of occurrence of the emergency, the magnitude of the impact, whether the vehicles has overturned or submerged, or whether any person has responded to a message, as required using the short-distance radio communication.

The doors are unlocked and the personal information is disclosed as in the case of the on-vehicle apparatus 1, described above. These operations may be performed only if an approaching person such as a rescuer is a well-intentioned rescuer; the personmaybe confirmed to be a rescue teammember, a police officer, or a guard from a security company with which a contract has been entered on the basis of an exterior image checked by the above exterior image monitoring section 92 or depending on whether or not the person has a predetermined RFID tag, or the person is confirmed not to be covering his or her face on the basis of the exterior image checked by the exterior imagemonitoring section 92 so that suspicious persons approaching the scene out of malice can be excluded. Alternatively, the doors may be unlocked with the personal information disclosed when, for example, the exterior image monitoring section 92 successfully acquires a face image of the rescuer.

If a member of the public approaches the scene of the accident and is determined not to be suspicious and not to be approaching the scene out of malice, the notification control section 302 of the on-vehicle apparatus 271 controls the communication section 292 so that information such as a network ID or a contact person ID can be transmitted to the rescuer's terminal apparatus 281 by short-distance communication such as Bluetooth (TM); the information such as a network ID or a contact person ID enables the party with which the terminal apparatus 281 communicates to determine that the holder of the terminal apparatus 281 is performing a rescue operation in an accident or the like. In this case, the monitoring server 283, emergency managing server 284, accident managing server 285, and client managing server 286 are configured to be able to receive information acquired by the terminal apparatus 281 having received the information such as the network ID or contact person ID. Then, if, for example, the rescuer's terminal apparatus 281 has an image pickup function, it can pick up images of the surroundings the image of which cannot be picked up by the camera in the vehicle involved in the accident. The terminal apparatus 281 can then transmit the images to the monitoring server 283, emergency managing server 284, accident managing server 285, and client managing server 286. This enables information on the scene of the accident to be provided to the police or firehouse early while preventing it from being disclosed to members of the public.

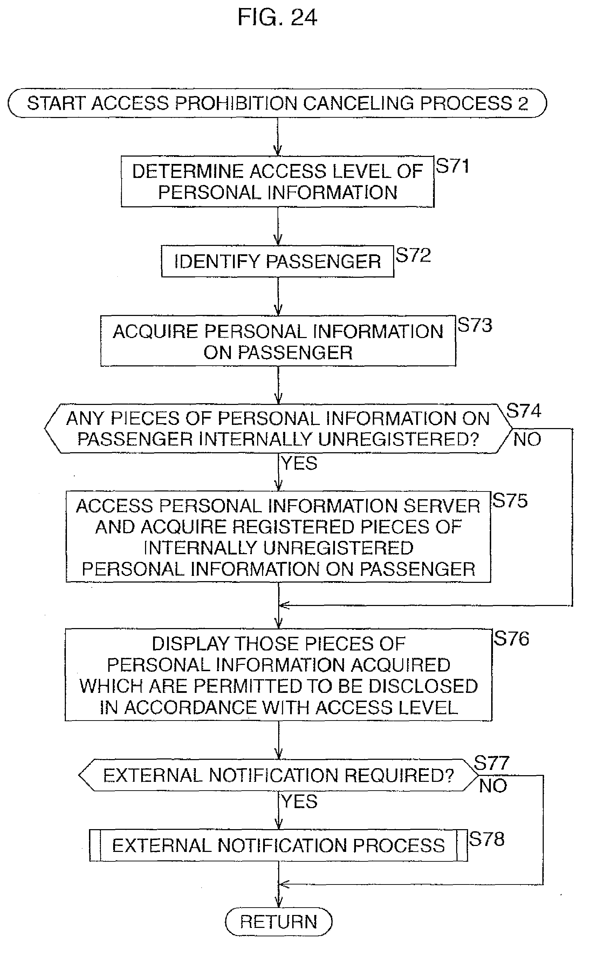

The process executed by the on-vehicle apparatus 271 is basically similar to the emergency monitoring process described with reference to FIG. 18. However, in step S12, an access prohibition cancelingprocess 2 in FIG. 24 is executed in place of the access prohibition canceling process 1 described with reference to FIG. 19.

The access prohibition canceling process 2 will be described with reference to the flowchart in FIG. 24.

In steps S71 to S73, processing is executed which is basically similar to that in steps S41 to S43 of the access prohibition canceling process 1 described with reference to FIG. 19.

That is, the access level of the personal information is determined on the basis of the determination of the emergency level by the emergency level determining section 125, and as required, on the basis of the operation input from a person requesting an access to the personal information which input is supplied by the operation input section 14. Each passenger is identified on the basis of the information supplied by the emergency level determining section 125. The acquisition of the personal information by the personal information acquiring section 127 is controlled on the basis of the determination of the access level of the personal information and the information on the passenger. The accessible personal information on the passenger is acquired from the personal information storing section 18 and supplied to the notification control section 302.

In step S74, the notification control section 302 determines whether or not any pieces of the personal information on the passenger are not internally registered, on the basis of the personal information supplied by the personal information acquiring section 127.

If the notification control section 302 determines in step S74 that any pieces of the personal information are internally unregistered, then in step S75, the notification control section 302 controls the communication section 292 to access the personal information registering server 282 via the radio packet communication network 251 and the Internet 252. If any of the internally unregistered pieces of the personal information on the passenger are registered in the personal information registering server 282, the notification control section 302 acquires and stores them in the personal information storing section 18.

If the notification control section 302 determines in step S74 that all of the personal information is internally registered or after the processing in step S75, it displays, in step S76, those pieces of the personal information supplied by the personal information acquiring section 127 or acquired from the personal information registering server 282 which are permitted to be accessed in accordance with the access level. Specifically, the notification control section 302 causes the display section 21 to sequentially display the passenger's face image, name, address, age, sex, and blood type, the medical information on the passenger, and the passenger's position in the car. The notification control section 302 may control the voice output section 22 to cause it to aurally communicate information that can be expressed in words.

In step S77, the notification control section 302 determines whether or not an external notification is required for at least one of the terminal apparatus 281, monitoring server 283, emergency managing server 284, accident managing server 285, and client managing server 286.

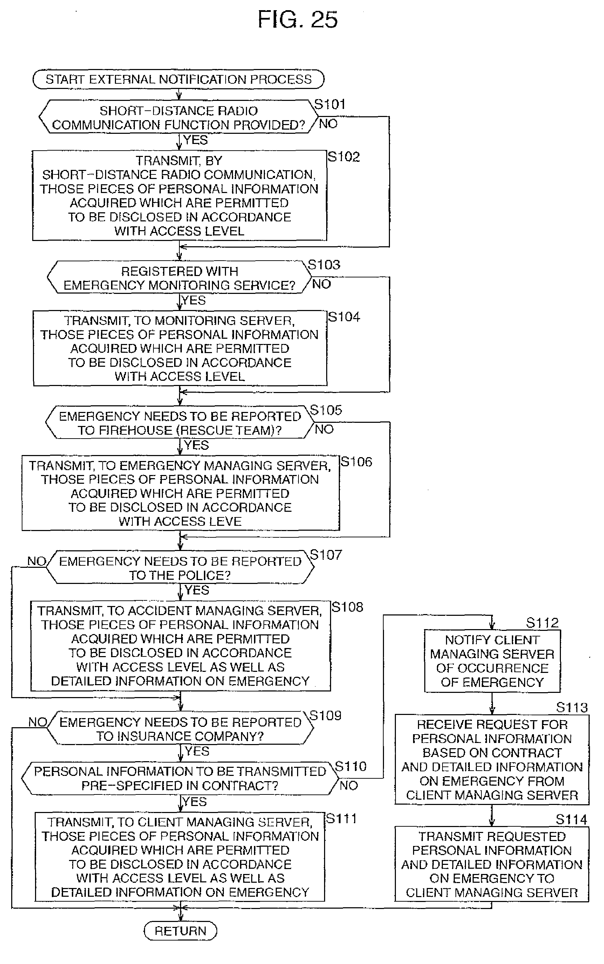

If the notification control section 302 determines in step S77 that an external notification is required, then in step S78, it executes an external notification process described later with reference to FIG. 25.

If the notification control section 302 determines in step S77 that no external notification is required or after the processing in step S78, the process returns to step S12 in FIG. 18 and then proceeds to step S13.

If this process cancels the access prohibition, the access level of the personal information is determined. The on-vehicle apparatus thus discloses only those pieces of the personal information on the person identified as a passenger which are suitable for the emergency which has occurred and not the unnecessary pieces of the personal information. The disclosable pieces of the personal information are communicated to any other destination as required. In this case, the access level of the disclosed information is also determined depending on the destination.

Now, with reference to the flowchart in FIG. 25, description will be given of the external notification process executed in step S78 in FIG. 24.

In step S101, the notification control section 302 determines whether or not the communication section 292 has a short-distance radio communication function.

If the notification control section 302 determines in step S101 that the communication section 292 has a short-distance radio communication function, then in step S102, it controls the communication section 292 to transmit, by short-distance radio communication, those pieces of the personal information acquired which are permitted to be disclosed in accordance with the access level.

If the notification control section 302 determines in step S101 that the communication section 292 does not have a short-distance radio communication function or after the processing in step S102, it determines in step S103, whether or not the on-vehicle apparatus 1 is registered with an emergency monitoring service.

If the notification control section 302 determines in step S103 that the non-vehicle apparatus 1 is registered with the emergencymonitoring service, then in step S104 it controls the communication section 292 to transmit those pieces of the personal information acquired which are permitted to be disclosed in accordance with the access level, to the monitoring server 283 via the radio packet communication network 251 and the Internet 252.

If the notification control section 302 determines in step S103 that the non-vehicle apparatus 1 is not registered with the emergency monitoring service or after the processing in step S104, it determines in step S105 whether or not the emergency needs to be reported to a firehouse (rescue team), on the basis of the operation input supplied by the operation input section 14 or the determination of the emergency level or personal information access level.

If the notification control section 302 determines in step S105 that the emergency needs to be reported to the firehouse (rescue team), then in step S106, it controls the communication section 292 to transmit those pieces of the personal information acquired which are permitted to be disclosed in accordance with the access level, to the emergency managing server 284 via the radio packet communication network 251 and the Internet 252.

If the notification control section 302 determines in step S105 that the emergency need not be reported to the firehouse (rescue team) or after the processing in step S106, it determines in step S107 whether or not the emergency needs to be reported to the police, on the basis of the operation input supplied by the operation input section 14 or the determination of the emergency level or personal information access level.

If the notification control section 302 determines in step S107 that the emergency needs to be reported to the police, then in step S108, it controls the communication section 292 to transmit those pieces of the personal information acquired which are permitted to be disclosed in accordance with the access level as well as the detailed information on the emergency, to the accident managing server 285 via the radio packet communication network 251 and the Internet 252.

If the notification control section 302 determines in step S107 that the emergency need not be reported to the police or after the processing in step S108, it determines in step S109 whether or not the emergency needs to be reported to the insurance company, on the basis of the operation input supplied by the operation input section 14 or the determination of the emergency level or personal information access level.

If the notification control section 302 determines in step S109 that the emergency needs to be reported to the insurance company, it determines in step S110 whether or not any pieces of the personal information to be transmitted to the insurance company are pre-specified in the contract.

If the notification control section 302 determines in step S110 that some pieces of the personal information to be transmitted to the insurance company are pre-specified in the contract, then in step S111, it controls the communication section 292 to transmit those pieces of the personal information acquired which is transmitted in accordance with the contract and are permitted to be disclosed in accordance with the access level as well as the detailed information on the emergency, to the client managing server 286 via the radio packet communication network 251 and the Internet 252.

If the notification control section 302 determines in step S110 that none of the personal information to be transmitted to the insurance company is pre-specified in the contract, then in step S112, it controls the communication section 292 to notify the client managing server 286 of the occurrence of the emergency via the radio packet communication network 251 and the Internet 252.

In step S113, the notification control section 302 receives a request for the personal information based on the contract and the detained information on the emergency which have been received by the communication section 292 from the client managing server 286 via the radio packet communication network 251 and the Internet 252.

In step S114, the notification control section 302 controls the communication section 292 to transmit the requested personal information and detailed information on the emergency to the client managing server 286 via the radio packet communication network 251 and the Internet 252.

If the notification control section 302 determines in step S109 that the emergency need not be reported to the insurance company or after the processing in step S111 or S114, the process returns to step S78 in FIG. 24, returns to step S12 in FIG. 18, and then proceeds to step S13.

This process allows the personal information permitted to be disclosed and the detailed information on the emergency to be transmitted in association with the destination of the personal information.

Thus, when a critical event such as an accident or a fire occurs, the present invention can make only the appropriate pieces of the information on the persons related to the event accessible to others. To disclose the personal information, the display section 21 may display face images and personal information or a voice output may be used. Alternatively, the information may be communicated to an external apparatus by short-distance radio communication or a network.

The on-vehicle apparatus 1 or 271, to which the present invention is applied, may be mounted at any position inside or outside the car, for example, near the doors, roof, or rear window or in the upper part of the trunk. However, since the on-vehicle apparatus 1 or 271 is assumed to be used after the occurrence of an accident, it is desirably mounted, if possible, at a position in the car compartment and close to the passengers which is unlikely to be affected by accidents. The on-vehicle apparatus 1 or 271 is also desirably configured so as not to be easily destroyed. The on-vehicle apparatus 1 or 271 may have any shape provided that it is mounted in a passenger car; it may be a car navigation apparatus mounted in the center of a console or a portable apparatus such as a PDA or a cellular phone. The on-vehicle apparatus 1 or 271, to which the present invention is applied, may be a part of equipment having functions for achieving a particular purpose as described with reference to FIGS. 7 to 17 or may share functions with some components of the equipment.

The on-vehicle apparatus 1 or 271, to which the present invention is applied, maybe entirely installed in one housing or divided into a plurality of pieces which are installed in the respective of housings. If the on-vehicle apparatus 1 or 271 is divided into a plurality of housings, information is transmitted among the housings via wired or wireless connections. Some or all of the arrangements for achieving the functions of the on-vehicle apparatus 1 or 271, to which the present invention is applied, may be configured as modules such as circuit boards which are incorporated into other apparatuses.

The on-vehicle apparatus 1 or 271, to which the present invention is applied, may authenticate persons by combining biological authentication and, for example, electronic authentication utilizing RFIDs or the like.

The above series of processes may be executed by software. Programs constituting the software and stored in recording media are installed in a computer integrated into dedicated hardware or, for example, a general-purpose personal computer that can execute various functions via various installed programs.

The recording media is provided separately from the computer as shown in FIG. 1 and 21. The recording media is composed of a magnetic disk (including a flexible disk), an optical disk (including a CD-ROM (Compact Disk-Read Only Memory) and a DVD (Digital Versatile Disk)), a magneto optic disk (including an MD (Mini-Disk) (trade mark)), or removable media 41 consisting of a semiconductor memory or the like. The programs are recorded on the recording media, which is then distributed to the user to provide him or her with the programs.

In the present specification, the steps of describing the programs recorded in the recording media include not only those executed in the described order in accordance with the time series but also those which are not necessarily executed in accordance with the time series but in parallel or individually.

In the present specification, the system corresponds to the entire equipment composed of a plurality of apparatuses.

**Claims**

1. An information processing apparatus comprising:

    authenticating means for authenticating a passenger in a vehicle;
    state managing means for managing a state of the vehicle;
    emergency determining means for determining whether or not the vehicle is in an emergency on the basis of the state of the vehicle managed by the state managing means;
    disclosed content determining means for determining a disclosed content of personal information on the passenger on the basis of the determination by the emergency determining means;
    personal information acquiring means for acquiring personal information on the passenger determined to be disclosed, on the basis of the determination by the disclosed content determining means; and
    disclosing means for disclosing the personal information on the passenger determined to be disclosed, which has been acquired by the personal information acquiring means.

2. The information processing apparatus according to claim 1, wherein the emergency determining means further determines the level of the emergency if the vehicle is in the emergency, and
    the disclosed content determining means determines the disclosed content of the personal information on the passenger on the basis of the determination of the level of the emergency by the emergency determining means.

3. The information processing apparatus according to claim 1, wherein the disclosed content determining means further determines the disclosed content of the personal information on the passenger on the basis of a person who is referencing the personal information on the passenger disclosed by the disclosing means.

4. The information processing apparatus according to claim 1, wherein the disclosed content determining means further determines the disclosed content of the personal information on the passenger on the basis of settings present when the personal information on the passenger is registered.

5. The information processing apparatus according to claim 1, wherein the disclosed content determining means further determines the disclosedcontent of the personal information on the passenger on the basis of the type of the vehicle.

6. The information processing apparatus according to claim 1, further comprising:

    response confirming means for confirming a response from the passenger,
    wherein the emergency determining means determines whether or not the vehicle is in the emergency on the basis of the state of the vehicle managed by the state managing means and the response from the passenger confirmed by the response confirming means.

7. The information processing apparatus according to claim 1, wherein the state managing means manages the state of the vehicle on the basis of information on the state of the vehicle acquired by a sensor provided in the vehicle.

8. The information processing apparatus according to claim 7, wherein the state managing means receives a supply of information indicative of the state of the vehicle acquired by a plurality of sensors provided in the vehicle,
    the emergency determining means determines whether or not an emergency is occurring on the basis of the state of the vehicle acquired by a plurality of the sensors.

9. The information processing apparatus according to claim 1, wherein the state managing means manages the state of the vehicle on the basis of an image of an interior or exterior of the vehicle acquired by image acquiring means provided in the vehicle.

10. The information processing apparatus according to claim 1, further comprising:

operation input means for receiving an input predetermined code number,
wherein the emergency determining means recognizes that the vehicle may have been stolen if the predetermined code number is input via the operation input means.

11. The information processing apparatus according to claim 1, further comprising:

display means for displaying information,
wherein the disclosing means controls display, on the display means, of the personal information on the passenger determined to be disclosed, which has been acquired by the personal information acquiring means.

12. The information processing apparatus according to claim 11, wherein the display means displays information containing a face image as personal information on the passenger.

13. The information processing apparatus according to claim 13, further comprising:

display means for displaying information,
wherein the authenticating means acquires a face image of the passenger and uses the face image to execute an authenticating process, and
the disclosing means controls the display, on the display means, of the personal information on the passenger determined to be disclosed, which has been acquired by the personal information acquiring means and the passenger's face image.

14. The information processing apparatus according to claim 1, further comprising:

display means for displaying information; and
intention recognizing means for acquiring and analyzing the passenger's face image to recognize the passenger's intention,
wherein the disclosing means controls the display, on the displaymeans, of the personal information on the passenger determined to be disclosed, which has been acquired by the personal information acquiring means and the passenger's face image acquired by the intention recognizing means.

15. The information processing apparatus according to claim 1, further comprising:

voice output means for outputting a voice,
wherein the disclosing means controls the notification, through the voice output means, of the personal information on the passenger determined to be disclosed, which has been acquired by the personal information acquiring means.

16. The information processing apparatus according to claim 1, further comprising:

communication means for controlling transmission and reception of information to and from other apparatuses,
wherein the disclosing means controls the transmission to the other apparatuses, by the communication means, of the personal information on the passenger determined to be disclosed, which has been acquired by the personal information acquiring means.

17. An information processing method for an information processing apparatus that manages personal information on a passenger in a vehicle, the method comprising:

an authenticating step of authenticating a passenger in the vehicle;
a state managing step of managing a state of the vehicle;
an emergency determining step of determining whether or not the vehicle is in an emergency on the basis of the state of the vehicle managed by processing in the state managing step;
a disclosed content determining step of determining a disclosed content of personal information on the passenger on the basis of the determination by processing in the emergency determining step;
a personal information acquiring step of acquiring personal information on the passenger determined to be disclosed, on the basis of the determination by processing in the disclosed content determining step; and
disclosing step of disclosing the personal information on the passenger determined to be disclosed, which has been acquired by processing in the personal information acquiring step.

**18.** A program for allowing a computer to execute a process of managing personal information on a passenger in a vehicle, the process comprising:

an authenticating step of authenticating the passenger in the vehicle;
a state managing step of managing a state of the vehicle;
an emergency determining step of determining whether or not the vehicle is in an emergency on the basis of the state of the vehicle managed by processing in the state managing step;
a disclosed content determining step of determining a disclosed content of personal information on the passenger on the basis of the determination by processing in the emergency determining step;
a personal information acquiring step of acquiring personal information on the passenger determined to be disclosed, on the basis of the determination by processing in the disclosed content determining step; and
disclosing step of disclosing the personal information on the passenger determined to be disclosed, which has been acquired by processing in the personal information acquiring step.

**19.** Recording media on which the program according to claim 18 is recorded.

**20.** An information processing system comprising:

an on-vehicle apparatus mounted in a vehicle; and
at least one information processing apparatus that transmits and receives information to and from the on-vehicle apparatus, the on-vehicle apparatus comprising:
authenticating means for authenticating a passenger in the vehicle;
state managing means for managing a state of the vehicle;
emergency determining means for determining whether on not the vehicle is in an emergency on the basis of the state of the vehicle managed by the state managing means;
disclosed content determining means for determining a disclosed content of personal information on the passenger on the basis of the determination by the emergency determining means;
personal information acquiring means for acquiring personal information on the passenger determined to be disclosed, on the basis of the determination by the disclosed content determining means;
disclosing means for disclosing the personal information on the passenger determined to be disclosed, which has been acquired by the personal information acquiring means; and
communication means that transmits and receives information to and from the information processing apparatus.

**21.** The information processing system according to claim 20, wherein if the personal information on the passenger is transmitted to the information processing apparatus by the communication means controlled by the disclosing means, the disclosed content determining means determines the disclosed content of the personal information on the passenger on the basis of the type of the information processing apparatus to which the personal information on the passenger is disclosed.

**22.** The information processing system according to claim 20, wherein the information processing apparatus stores the personal information on the passenger, and
the personal information acquiring means controls the communication means to acquire the personal information on the passenger from the information processing apparatus.

FIG. 1

ON-VEHICLE APPARATUS

- 16 AUTHENTICATION PROCESSING SECTION
- 15 BIOLOGICAL INFORMATION ACQUIRING SECTION
- 19 EMERGENCY DETECTING SECTION
- 20 EXTERNAL OPERATION SECTION
- 23 COMMUNICATION SECTION
- 14 OPERATION INPUT SECTION
- 21 DISPLAY SECTION

31 BUS

- 24 DRIVE
- 11 CPU
- 12 ROM
- 13 RAM
- 17 AUTHENTICATION RESULT STORING SECTION
- 18 PERSONAL INFORMATION STORING SECTION
- 22 VOICE OUTPUT SECTION

REMOVABLE MEDIA ~41

EP 1 770 572 A2

FIG. 2

## FIG. 3

75〜 REGISTER    CHANGE    DELETE 〜77
                    〜76

◁          ▷

NAME [                    ] □

ADDRESS [                    ] □

DATE OF BIRTH [          ]    SEX ◎MALE ○FEMALE □

FACE

BLOOD TYPE [      ] DRIVER'S LICENSE NUMBER [        ] □

ID CODE [        ] E-MAIL ADDRESS [        ] □

MEDICAL INFORMATION [                    ] □

PERSON TO COMMUNICATE WITH IN EMERGENCY [                    ] □

ADDRESS OF PERSON TO COMMUNICATE IN WITH EMERGENCY [                    ] □

CONTRACT NUMBER [      ] CONTRACT TYPE [        ] □

PLACE OF WORK [                    ] □

TELEPHONE NUMBER OF PLACE OF WORK [                    ] □

72 PASSENGER POSITION DISPLAY AREA    73 PERSONAL INFORMATION INPUT BOX    74 DISCLOSED GROUP SETTING BOX

71 FACE IMAGE DISPLAY AREA

## FIG. 4

19

EMERGENCY DETECTING SECTION

91 — EXTERIOR IMAGE MONITORING SECTION

92 — INTERIOR IMAGE MONITORING SECTION

93 — VEHICLE STATE MONITORING SECTION

94 — ACCELERATION SENSOR

95 — ANGULAR SPEED SENSOR

96 — COLLISION SENSOR

97 — TEMPERATURE SENSOR

98 — SUBMERGENCE SENSOR

100

SENSOR CONNECTING SECTION

## FIG. 5

STATE MANAGING SECTION 122

AUTHENTICATION RESULT ACQUIRING SECTION 121

RESPONSE PROCESSING SECTION ~123

EMERGENCY LEVEL DETERMINING SECTION 125

EXTERNAL OPERATION CONTROL SECTION 128

PERSONAL INFORMATION ACCESS LEVEL DETERMINING SECTION ~126

NOTIFICATION CONTROL SECTION 124

PERSONAL INFORMATION ACQUIRING SECTION ~127

EP 1 770 572 A2

# FIG. 6

| | | |
|---|---|---|
| ◁ | STOP | ▷ |

FACE

NAME — TARO YAMADA

ADDRESS

DATE OF BIRTH — SEX ◉MALE ○FEMALE

BLOOD TYPE — DRIVER'S LICENSE NUMBER

ID CODE — E-MAIL ADDRESS

MEDICAL INFORMATION

PERSON TO COMMUNICATE WITH IN EMERGENCY

ADDRESS OF PERSON TO COMMUNICATE IN WITH EMERGENCY

146 PERSONAL INFORMATION DISPLAY AREA

DISPLAY END

147 DISPLAY END BUTTON

142 PASSENGER POSITION DISPLAY AREA

143 INTERIOR IMAGE DISPLAY AREA

141 FACE IMAGE DISPLAY AREA

144 FRONT IMAGE DISPLAY AREA

145 REAR IMAGE DISPLAY AREA

EP 1 770 572 A2

FIG. 7

INTENTION CHECKING APPARATUS — 201

```
                                                                MOVEMENT
                                                                RECOGNIZING — 216
                                                                SECTION

                                              214                NODDING — 221
                           213                FACE DIRECTION    RECOGNIZING
  VOICE                    CAMERA             SENSING           SECTION
  OUTPUT — 211                                SECTION                          INTENTION — 217
  SECTION                                                                      RECOGNIZING
                                                                              SECTION
                                              LINE-OF-SIGHT     HEAD SHAKE
  SPEAKER — 212                               DIRECTION         RECOGNIZING
                                              SENSING SECTION   SECTION
                                                  215               222
```

EP 1 770 572 A2

36

## FIG. 8

----> LINE-OF-SIGHT DIRECTION

----> FACE DIRECTION

## FIG. 9

## FIG. 10

----> LINE-OF-SIGHT DIRECTION

----> FACE DIRECTION

## FIG. 11

# FIG. 12

UPWARD FACE DIRECTION

DOWNWARD
LINE-OF
-SIGHT
DIRECTION

0   5        30

UPWARD
LINE-OF
-SIGHT
DIRECTION

-5

-30

DOWNWARD FACE DIRECTION

## FIG. 13

- - - -> LINE-OF-SIGHT DIRECTION
———> FACE DIRECTION

## FIG. 14

## FIG. 15

- - - -> LINE-OF-SIGHT DIRECTION

———> FACE DIRECTION

## FIG. 16

# FIG. 17

RIGHTWARD FACE DIRECTION

R

30

5

LEFTWARD
LINE-OF
-SIGHT
DIRECTION

-30          -5    0    5         30

RIGHTWARD
LINE-OF
-SIGHT
DIRECTION

-5

-30

L

LEFTWARD FACE DIRECTION

# FIG. 18

```
( START EMERGENCY MONITORING PROCESS )
                      │
                      ▼
        ┌─────────────────────────────────┐ S1
        │ ACQUIRE AUTHENTICATION RESULT   │
        └─────────────────────────────────┘
                      │
                      ▼
        ┌─────────────────────────────────┐ S2
        │ ACQUIRE IMAGE PROCESSING RESULTS AND │
        │ SENSOR INPUTS REQUIRED TO DETECT EMERGENCY │
        └─────────────────────────────────┘
                      │
           NO ◄─< EMERGENCY DETECTED? > S3
                      │ YES
    NO ◄─< OPERATION INDICATING  S4
           EMERGENCY INPUT? >
                      │ YES
                      ▼
        ┌─────────────────────────────┐ S5
        │ OUTPUT RESPONSE MESSAGE     │
        └─────────────────────────────┘
                      │
           < ANY RESPONSE? > S6
                 │        │ NO
              YES▼        ▼
        ┌──────────────┐ S10   ┌──────────────┐ S7
        │ DETERMINE    │       │ DETERMINE    │
        │ EMERGENCY    │       │ EMERGENCY    │
        │ LEVEL        │       │ LEVEL        │
        └──────────────┘       └──────────────┘
                                     │
                         < CONDITIONS FOR  S8
                           PERMITTING ACCESSE
                           TO PERSONAL
                           INFORMATION MET? >──YES──► (1)
                                     │ NO
        < CAN INTENTION TO PREVENT  S11
          ACCESSES TO PERSONAL INFORMATION
          FROM BEING PERMITTED
          BE CONFIRMED? >──YES─┐
                      │ NO      ▼
  (1)                          ┌──────────────┐ S9
   │                           │ CONTINUE     │
   │         ┌──────────────┐  │ ACCESS       │
   └────────►│ ACCESS PROHIBITION S12 │ PROHIBITED │
             │ CANCELING PROCESS │   │ STATE        │
             └──────────────┘       └──────────────┘
                      │
        NO ◄─< EXTERNAL OPERATION REQUIRED? > S13
                      │ YES
                      ▼
        ┌─────────────────────────────┐ S14
        │ CONTROL EXTERNAL OPERATION  │
        │ ON THE BASIS OF EMERGENCY LEVEL │
        └─────────────────────────────┘
                      │
  NO ◄─< EMERGENCY DETERMINATION TO BE CANCELLED? > S15
                      │ YES
                      ▼
        ┌─────────────────────────────┐ S16
        │ CANCEL EMERGENCY DETERMINATION │
        └─────────────────────────────┘
                      │
  NO ◄─< MONITORING OF EMERGENCY TO BE ENDED? > S17
                      │ YES
                      ▼
                   ( END )
```

41

# FIG. 19

```
( START ACCESS PROHIBITION CANCELING PROCESS 1 )
                          │
                          ▼
        ┌──────────────────────────────┐
        │ DETERMINE ACCESS LEVEL OF     │ S41
        │ PERSONAL INFORMATION          │
        └──────────────────────────────┘
                          │
                          ▼
            ┌──────────────────────┐
            │ IDENTIFY PASSENGER    │ S42
            └──────────────────────┘
                          │
                          ▼
       ┌────────────────────────────────┐
       │ ACQUIRE PERSONAL INFORMATION    │ S43
       │ ON PASSENGER                    │
       └────────────────────────────────┘
                          │
                          ▼
     ┌──────────────────────────────────────┐
     │ DISPLAY THOSE PIECES OF               │ S44
     │ PERSONAL INFORMATION ACQUIRED         │
     │ WHICH IS PERMITTED TO BE DISCLOSED    │
     │ IN ACCORDANCE WITH ACCESS LEVEL       │
     └──────────────────────────────────────┘
                          │
                          ▼
                    ( RETURN )
```

FIG. 20

283

284

285

286

MONITORING
SERVER

EMERGENCY
MANAGING SERVER

ACCIDENT
MANAGING SERVER

CLIENT
MANAGING SERVER

PERSONAL INFORMATION
REGISTERING SERVER

INTERNET

~252

282

RADIO PACKET
COMMUNICATION NETWORK

~251

ON-VEHICLE
APPARATUS

SHORT-DISTANCE
COMMUNICATION

TERMINAL
APPARATUS

271

281

EP 1 770 572 A2

# FIG. 21

EP 1 770 572 A2

# FIG. 22

EP 1 770 572 A2

# FIG. 23

321 ~ SECURITY COMPANY
POLICE — 322
FIREHOUSE — 323

FACE

STOP

NAME — TARO YAMADA
ADDRESS
DATE OF BIRTH — SEX ⊚ MALE ○ FEMALE
BLOOD TYPE — DRIVER'S LICENSE NUMBER
ID CODE — E-MAIL ADDRESS
MEDICAL INFORMATION
PERSON TO COMMUNICATE WITH IN EMERGENCY
ADDRESS OF PERSON TO COMMUNICATE IN WITH EMERGENCY

146 PERSONAL INFORMATION DISPLAY AREA

DISPLAY END — 147 DISPLAY END BUTTON

142 PASSENGER POSITION DISPLAY AREA

143 INTERIOR IMAGE DISPLAY AREA

141 FACE IMAGE DISPLAY AREA

144 FRONT IMAGE DISPLAY AREA

145 REAR IMAGE DISPLAY AREA

EP 1 770 572 A2

## FIG. 24

```
( START ACCESS PROHIBITION CANCELING PROCESS 2 )
                          │
                          ▼
        ┌─────────────────────────────────┐ S71
        │ DETERMINE ACCESS LEVEL OF       │
        │ PERSONAL INFORMATION            │
        └─────────────────────────────────┘
                          │
                          ▼
            ┌─────────────────────┐ S72
            │ IDENTIFY PASSENGER  │
            └─────────────────────┘
                          │
                          ▼
        ┌─────────────────────────────────┐ S73
        │ ACQUIRE PERSONAL INFORMATION    │
        │ ON PASSENGER                    │
        └─────────────────────────────────┘
                          │
                          ▼
        ╱ ANY PIECES OF PERSONAL INFORMATION ON ╲ S74
        ╲ PASSENGER INTERNALLY UNREGISTERED?    ╱ NO
                          │ YES
                          ▼
        ┌─────────────────────────────────────┐ S75
        │ ACCESS PERSONAL INFORMATION SERVER  │
        │ AND ACQUIRE REGISTERED PIECES OF    │
        │ INTERNALLY UNREGISTERED             │
        │ PERSONAL INFORMATION ON PASSENGER   │
        └─────────────────────────────────────┘
                          │
                          ▼
        ┌─────────────────────────────────────┐ S76
        │ DISPLAY THOSE PIECES OF             │
        │ PERSONAL INFORMATION ACQUIRED       │
        │ WHICH ARE PERMITTED TO BE DISCLOSED │
        │ IN ACCORDANCE WITH ACCESS LEVEL     │
        └─────────────────────────────────────┘
                          │
                          ▼
        ╱ EXTERNAL NOTIFICATION REQUIRED? ╲ S77
        ╲                                 ╱ NO
                          │ YES
                          ▼
        ┌─────────────────────────────────┐ S78
        │ EXTERNAL NOTIFICATION PROCESS   │
        └─────────────────────────────────┘
                          │
                          ▼
                   ( RETURN )
```

# FIG. 25

START EXTERNAL NOTIFICATION PROCESS

S101 SHORT-DISTANCE RADIO COMMUNICATION FUNCTION PROVIDED? NO

YES

S102 TRANSMIT, BY SHORT-DISTANCE RADIO COMMUNICATION, THOSE PIECES OF PERSONAL INFORMATION ACQUIRED WHICH ARE PERMITTED TO BE DISCLOSED IN ACCORDANCE WITH ACCESS LEVEL

S103 REGISTERED WITH EMERGENCY MONITORING SERVICE? NO

YES

S104 TRANSMIT, TO MONITORING SERVER, THOSE PIECES OF PERSONAL INFORMATION ACQUIRED WHICH ARE PERMITTED TO BE DISCLOSED IN ACCORDANCE WITH ACCESS LEVEL

S105 EMERGENCY NEEDS TO BE REPORTED TO FIREHOUSE (RESCUE TEAM)? NO

YES

S106 TRANSMIT, TO EMERGENCY MANAGING SERVER, THOSE PIECES OF PERSONAL INFORMATION ACQUIRED WHICH ARE PERMITTED TO BE DISCLOSED IN ACCORDANCE WITH ACCESS LEVE

S107 EMERGENCY NEEDS TO BE REPORTED TO THE POLICE? NO

YES

S108 TRANSMIT, TO ACCIDENT MANAGING SERVER, THOSE PIECES OF PERSONAL INFORMATION ACQUIRED WHICH ARE PERMITTED TO BE DISCLOSED IN ACCORDANCE WITH ACCESS LEVEL AS WELL AS DETAILED INFORMATION ON EMERGENCY

S109 EMERGENCY NEEDS TO BE REPORTED TO INSURANCE COMPANY? NO

YES

S110 PERSONAL INFORMATION TO BE TRANSMITTED PRE-SPECIFIED IN CONTRACT? NO

YES

S111 TRANSMIT, TO CLIENT MANAGING SERVER, THOSE PIECES OF PERSONAL INFORMATION ACQUIRED WHICH ARE PERMITTED TO BE DISCLOSED IN ACCORDANCE WITH ACCESS LEVEL AS WELL AS DETAILED INFORMATION ON EMERGENCY

S112 NOTIFY CLIENT MANAGING SERVER OF OCCURRENCE OF EMERGENCY

S113 RECEIVE REQUEST FOR PERSONAL INFORMATION BASED ON CONTRACT AND DETAILED INFORMATION ON EMERGENCY FROM CLIENT MANAGING SERVER

S114 TRANSMIT REQUESTED PERSONAL INFORMATION AND DETAILED INFORMATION ON EMERGENCY TO CLIENT MANAGING SERVER

RETURN

**REFERENCES CITED IN THE DESCRIPTION**

**Patent documents cited in the description**

- JP 2003108945 A

- JP 2003162578 A